(19)

Europäisches Patentamt

European Patent Office

Office européen des brevets

(11)  **EP 4 530 353 A1**

(12)  **EUROPEAN PATENT APPLICATION**

(43) Date of publication:
**02.04.2025   Bulletin 2025/14**

(21) Application number: **23200346.7**

(22) Date of filing: **28.09.2023**

(51) International Patent Classification (IPC):
**C12N 15/86** (2006.01)

(52) Cooperative Patent Classification (CPC):
**C12N 15/86;** C12N 2740/00051; C12N 2740/16011;
C12N 2750/00051; C12N 2750/14111

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sartorius Xell GmbH
33758 Schloß Holte-Stukenbrock (DE)**

(72) Inventors:
• **Krämer, Jan Niklas
  33758 Schloss Holte-Stukenbrock (DE)**

• **Teschner, Kathrin
  33758 Schloss Holte-Stukenbrock (DE)**
• **Buve, Alyssa
  33758 Schloss Holte-Stukenbrock (DE)**
• **Vetter, Luisa
  33758 Schloss Holte-Stukenbrock (DE)**

(74) Representative: **Rose, Jonas Christopher
  Sartorius Stedim Biotech GmbH
  Intellectual Property Management
  August-Spindler-Straße 11
  37079 Göttingen (DE)**

(54)   **METHODS, SYSTEMS, COMPOSITIONS AND USES FOR IMPROVED PRODUCTION OF VIRAL
VECTORS**

(57)   The present invention provides methods for more efficient production of a viral vector which can be used for gene therapy. Specifically, the invention relates to culturing mammalian cells in the presence of compounds enhancing the viral vector production. Also provided are advantageous systems, compositions, and uses of the compounds for increased viral vector production.

Figure 1

Mammalian cell in different bioprocess scales → Transient transfection → Add enhancer → Increased production of viral vector → Several fold increase in yield

**EP 4 530 353 A1**

Description

**TECHNICAL FIELD OF THE INVENTION**

The present invention provides methods for more efficient production of a viral vector which can be used for gene therapy. Specifically, the invention relates to culturing mammalian cells in the presence of compounds enhancing the viral vector production. Also provided are advantageous systems, compositions, and uses of the compounds for increased viral vector production.

**BACKGROUND OF THE INVENTION**

Gene therapy allows for using nucleic acids to repair malfunctioning DNA sequences or to introduce a compensatory change that will restore the normal physiological functions of the cell. Viral vectors provide an efficient means for modification of eukaryotic cells, and their use is commonplace in academic laboratories and industry for both research and clinical gene therapy applications. Gene therapy via gamma retroviruses, lentiviruses, adenoviruses, and adeno-associated viruses (AAV) is attractive because of the natural ability of viruses to enter and deliver genetic material to cells. Gammaretroviruses and lentiviruses are subtypes of retroviruses, which contain an RNA genome that is converted to DNA in the transduced cell by a virally encoded enzyme called reverse transcriptase.

Recombinant AAV-based gene transfer vectors are generated by utilizing the capsid protein shell of the wildtype virus but replacing the ssDNA genome of the natural virus with a therapeutic transgene cassette, retaining only the viral inverted terminal repeats (ITRs). In the absence of the rep proteins, ITR-flanked transgenes encoded by rAAV can form circular concatemers that persist as episomes in the nucleus of transduced cells. Because recombinant episomal DNA does not integrate into the host genome, rAAV is ideal for gene therapy applications and AAV-vector-mediated gene delivery and was recently approved e.g. for the treatment of inherited blindness and spinal muscular atrophy, and long-term therapeutic effects have been achieved for other rare diseases, including hemophilia and Duchenne muscular dystrophy. Also, the European Medicines Agency approved HEMGENIX by CSL Behring GmbH, Roctavian by BioMarin International Limited and Upstaza by PTC Therapeutics International Limited as AAV based gene therapies, underscoring its increasing importance. As a vector, AAV has many advantages, such as a negligible rate of integration into the host cell genome, no association with any human disease, and the inability to replicate without a helper virus. Set against this, lentiviral (LV) vectors are used in gene therapy applications due to their ability to stably integrate their genome into the host cell allowing long term treatments with single dosages.

In most instances, advanced therapeutics such as AAV- and lentivirus-derived vectors are produced in mammalian cell lines. A considerable advantage that especially the mammalian cell lines, e.g. such as those of human origin, share is their ability to confer certain post-translational modification (PTM) to the vector capsid. These PTM can affect the stability, infectivity and immunogenicity *in vivo,* thereby making them a crucial quality parameter.

In the past, a growing number of authors presented different approaches towards process optimization in viral vector production. Apart from the classic parameters of a biotechnological process such as pH, dissolved oxygen and carbon dioxide level and temperature, the process of transfection was optimized, e.g., including the cell density at the time of transfection, amount of plasmid DNA per cell, the molar ratio of the plasmids, and the transfection reagent. By optimizing these parameters, improvements in viral vector yield have already been achieved. In addition, the use of auxiliary compounds, for example in the form of small molecule enhancers, has already been proven to be effective in increasing recombinant protein yield. One enhancer is sodium butyrate, which is involved in the modulation of condensation of specific regions of DNA in the host cell genome, thereby influencing global gene expression. Other examples of enhancers of recombinant protein expression encompass valproic acid and dimethyl sulfoxide (DMSO).

Despite these improvements, there is still a need for more efficient viral vector production. Specifically, despite many years of research, the manufacture and production of sufficient quantities of viral vectors, such as lentiviral or AAV vectors, in mammalian cell culture, is still a major bottleneck in biotechnological processes. Expression of these viral vectors is mainly performed transiently or in some cases by stably integrating the genes required for viral vector production into the host genome. Especially the production of viral vectors by transient transfection is comparably inefficient, labor intensive and requires complex downstream processing to achieve the necessary quantity and quality for use in clinical trials. Furthermore, the scope of AAV- and lentivirus-based therapies is expanding at an increasing rate, so is the population of potential patients and thus, the necessity for improved viral vector production.

Therefore, it is an object of the present invention to increase the efficiency of viral vector production processes. In particular, it is an object to improve the viral vector production in cell culture by increasing the yield of viral vector productions, as well as viral vector quality.

## SUMMARY OF THE INVENTION

The present invention addresses the above-described needs. The present invention provides viral vector production methods, comprising providing a mammalian cell configured to produce a viral vector, followed by contacting the mammalian cells with a compound (herein also referred to as "enhancer" or "small molecule enhancer") to produce a viral vector. The inventors surprisingly identified that a flavonoid and/or M344 upon addition increased the production of viral vectors, such as AAV and lentivirus, by a mammalian cell. Specifically, the genomic titer but also the transducing titer of the viral vectors was markedly increased, improving the yield but also quality of the resulting viral vectors. The inventors observed these advantageous properties of the identified enhancers for different cell types but also different cultivation methods and scales supporting the robustness of the technology.

According to a first aspect, a viral vector production method is provided, comprising following steps:

(a) providing a mammalian cell configured to produce a viral vector;
(b) contacting the mammalian cell with a flavonoid and/or M344;
(c) culturing the mammalian cell in presence of the flavonoid and/or M344 to produce a viral vector; and
(d) optionally, harvesting the viral vector.

The method according to the first aspect advantageously allows for improved viral vector production. Specifically, contacting the mammalian cell with a flavonoid and/or M344 enhances viral vector production, which can result in an increase in genomic viral vector titer, as well as transducing viral vector titer. As a result, the yield of produced viral vector that is applicable for gene therapy is higher and of higher quality.

According to a second aspect, a viral vector production system is provided, comprising:

- a mammalian cell configured to transiently or stably produce a viral vector, preferably the mammalian cell is a HEK293 cell or a derivative of a HEK293 cell; and
- a flavonoid and/or M344; and
- a cell culture medium for culturing the mammalian cell.

According to a third aspect, a viral vector production composition is provided, comprising:

- a mammalian cell configured to transiently orstably produce a viral vector, preferably the mammalian cell is a HEK293 cell or a derivative of a HEK293 cell; and
- a flavonoid and/or M344;

wherein the flavonoid and/or M344 increases production of the genomic viral vector titer and/or transducing viral vector titer compared to a composition without the flavonoid and/or M344.

According to a fourth aspect, a flavonoid for use for increased transient or stable viral vector production is provided.

Further aspects of the invention are disclosed below. Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

## BRIEF DESCRIPTION OF THE FIGURES

Some exemplary embodiments of the present disclosure will now be described with reference to the accompanying drawings.

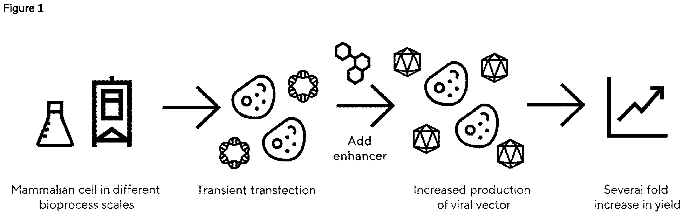

Fig. 1 shows a schematic overview of a viral vector production method using an enhancer, wherein the mammalian cell is modified by transiently transfecting the mammalian cell with one or more plasmids for viral vector production allowing for transient viral vector production. After or during transfection, the mammalian cell, which is capable of producing a viral vector, is contacted with an enhancer and cultured in order to increase the viral vector production and obtain a higher yield.

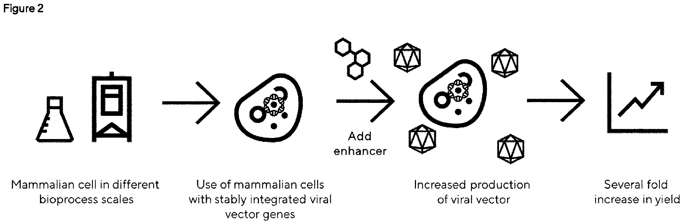

Fig. 2 shows a schematic overview of a viral vector production method using an enhancer, wherein the mammalian cell is modified by genome editing to include one or more nucleic acid molecules for viral vector production allowing for stable viral vector production (also referred to as stable producer cell). Such modified mammalian cell, which is capable of producing a viral vector, is contacted with an enhancer and cultured in order to increase the viral vector production and obtain a higher yield.

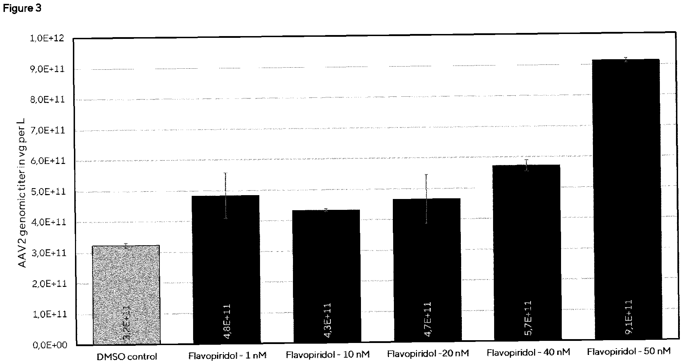

Fig. 3    shows AAV2 genomic titers in cell lysate 72 h after transient transfection in 24-well plates. In this case, FreeStyle 293-F cells were transfected in 24-well plates and flavopiridol was added 5 hours after transfection in the specified concentrations. Afterwards, the cells were incubated for 72 h in 24-well plates. DMSO control received 3μL DMSO (solvent used for flavopiridol), corresponding to 0.6 % of total culture volume. Quantification was performed on a QX200 ddPCR system (Bio-Rad) as described in the examples below. Shown are means ± MAD with n = 2.

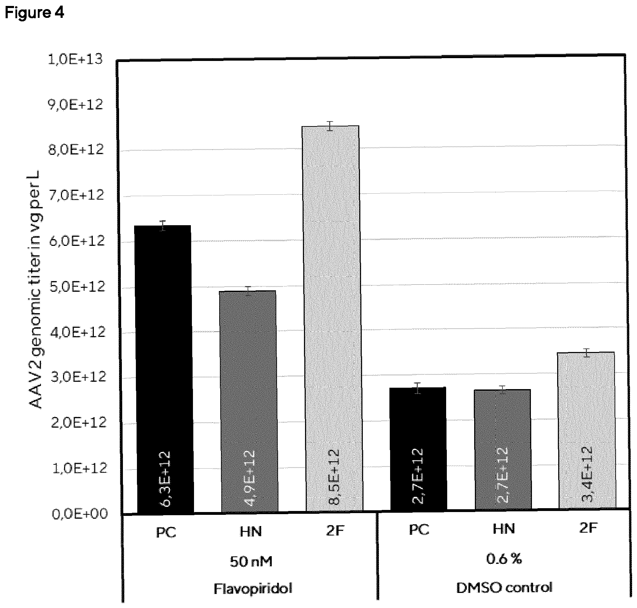

Fig. 4    shows AAV2 genomic titers in cell lysate 72 h after transient transfection in 125 mL-shake flasks. Viral Production Cells 2.0 (PC), a suspension adapted HEK293 clone (HN), and FreeStyle 293-F cells (2F) were transfected in 125 mL-shake flasks and flavopiridol was added 5 hours after transfection in the specified concentrations. DMSO control received 180 μL DMSO corresponding to 0.6 % of total culture volume. Afterwards, the cells were incubated for 72 h in 125 mL-shake flasks. Quantification was performed on a QX200 ddPCR system (Bio-Rad) as described in the examples below. Shown are values ± 95 % CI as calculated by the QX Manager software (Bio-Rad).

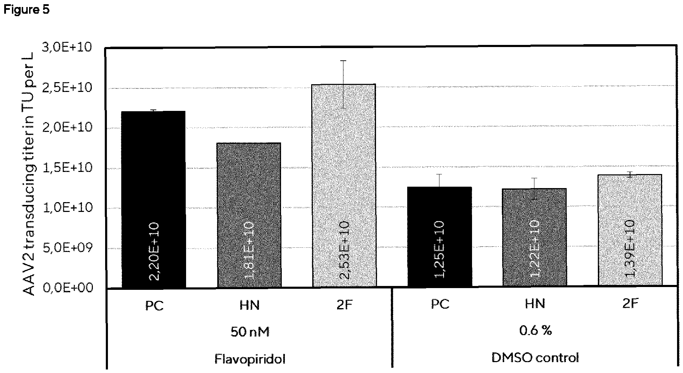

Fig. 5    shows AAV2 transducing titers of harvested cell lysate 72 h after transient transfection produced in 125 mL-shake flasks. Transduction was performed as described in the examples below. In brief, 100,000 cells in 1 mL of DMEM (+10 % FCS, + 2 mM L-glutamine) of adherent HEK293 were seeded in 12-well plates. 10 μL of either the undiluted or diluted sample of AAV-containing cell lysate was added to each well and cells were incubated for 72 h at 37 °C and 5 % CO2. Flow cytometry analysis was performed on an iQue® 3 flow cytometer (Sartorius AG) as described in the examples below. Shown are means ± MAD with n = 2.

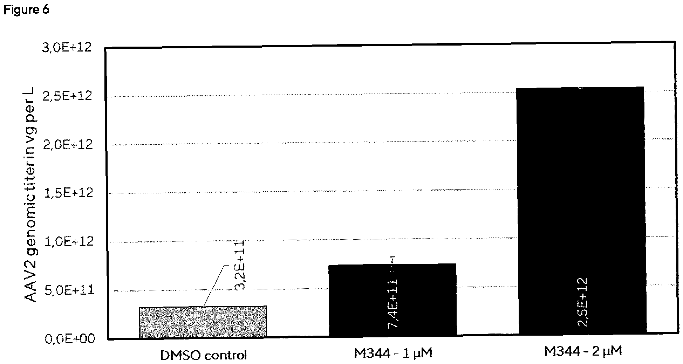

Fig. 6    shows AAV2 genomic titers in cell lysate 72 h after transient transfection in 24-well plates. Free-Style 293-F cells were transfected in 24-well plates and M344 was added 5 hours after transfection in the specified concentrations. DMSO control received 3 μL DMSO (solvent used for M344), corresponding to 0.6 % of total culture volume. Quantification was performed on a QX200 ddPCR system (Bio-Rad) as described in the examples below. Shown are means ± MAD with n = 2.

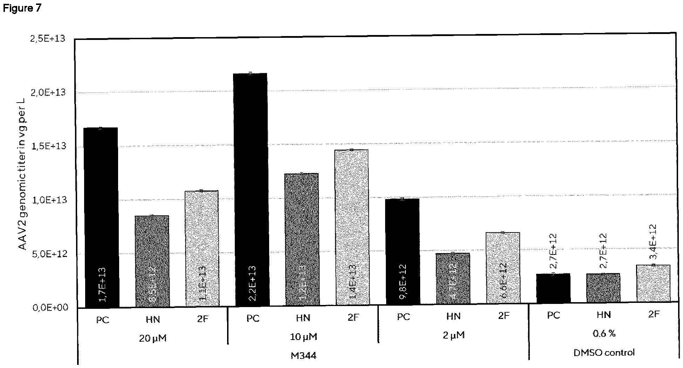

Fig. 7    shows AAV2 genomic titers in cell lysate 72 h after transient transfection in 125 mL-shake flasks. Viral Production Cells 2.0 (PC), the suspension-adapted HEK293 clone (HN) and FreeStyle 293-F cells (2F) were transfected in 125 mL-shake flasks and M344 was added 5 hours after transfection in the specified concentrations. DMSO control received 180 μL DMSO (solvent used for M344), corresponding to 0.6 % of total culture volume. Afterwards, the cells were incubated for 72 h in 125 mL-shake flasks. Quantification was performed on a QX200 ddPCR system (Bio-Rad) as described in the examples below. Shown are values ± 95 % CI as calculated by the QX Manager software (Bio-Rad).

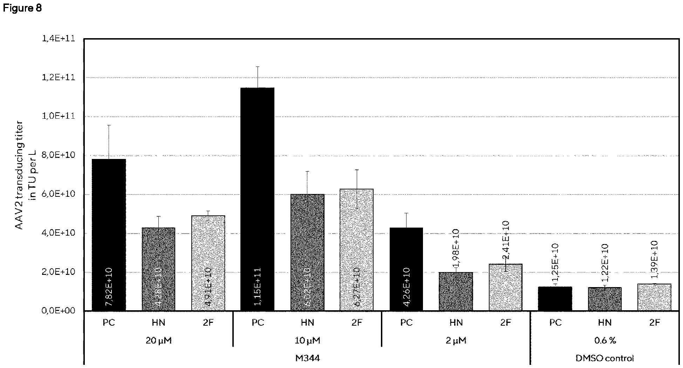

Fig. 8    shows AAV2 transducing titers of harvested cell lysate 72 h after transient transfection produced in 125 mL-shake flasks. Transduction was performed as described in the examples below. In brief, 100,000 adherent HEK293 cells were seeded in 12-well plates in 1 mL of DMEM (+10 % FCS, + 2 mM L-glutamine). 10 μL of either the undiluted or diluted sample of AAV-containing cell lysate was added to each well and cells were incubated for 72 h at 37 °C and 5 % CO2. Flow cytometry analysis was performed on an iQue® 3 flow cytometer (Sartorius AG) as described in the examples below. Shown are means ± MAD with n = 2.

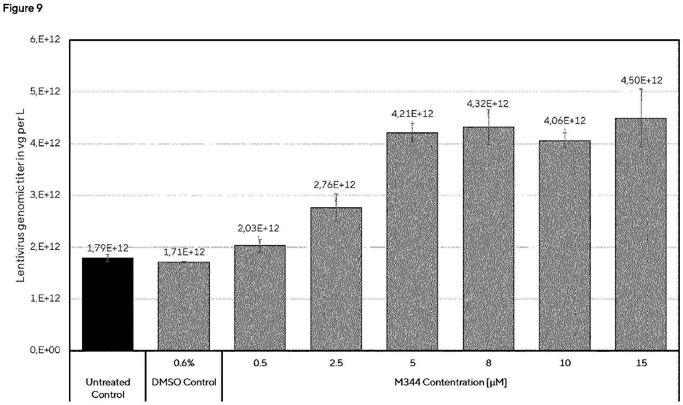

Fig. 9    shows LV genomic titers 72 h after transient transfection in 125 mL-shake flasks. LV MAX Production cells were transfected in 125 mL-shake flasks and M344 was added 4 to 6 hours after transfection in the specified concentrations. DMSO control received 180 μL DMSO (solvent used for M344), corresponding to 0.6 % of total culture volume. Quantification was performed on a QX200 ddPCR system as described in the examples below. Shown are means ± 95 % CI as calculated by the QX Manager software (Bio-Rad).

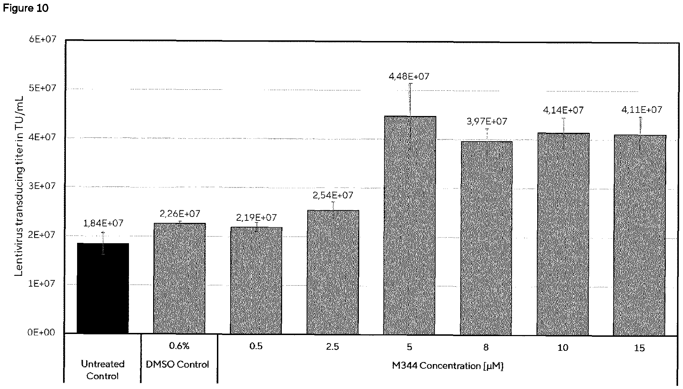

Fig. 10   shows LV transducing titers 72 h after harvest of the transient transfection in 125 mL-shake flasks. Transduction assay was performed as described in the Examples below. In brief, 100,000 cells in 1 mL of DMEM/F12 (+5 % FCS, + 6 mM L-glutamine, + 1.35 g/L glucose) of adherent HEK293FT containing 8 μg/mL hexadimethrine bromide were seed-ed in 12-well plates. 10 μL of either the undiluted or diluted sample was added to each well and cells were incubated for 72 h at 37 °C and 5 % CO2. Media change was performed after 24 h to wash out hexadimethrine bromide. Flow cytometry analysis was performed on an iQue® 3 flow cytometer (Sartorius AG). Shown are means ± MAD with n = 2.

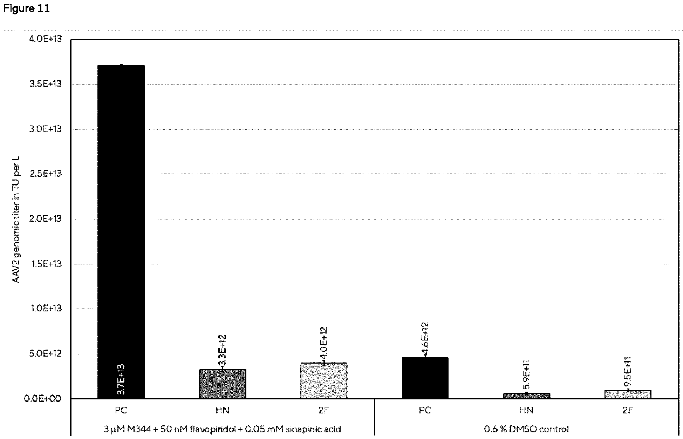

Fig. 11   shows AAV2 genomic titers in cell lysate 72 h after transient transfection in 125 mL-shake flasks. Viral Production Cells 2.0 (PC), the suspension-adapted HEK293 clone (HN) and FreeStyle 293-F cells (2F) were transfected in 125 mL-shake flasks and 50 nM flavopiridol, 3 μM M344 and 0.05 mM sinapinic acid were added 5 hours after transfection in the specified concentrations. DMSO control received 180 μL DMSO (solvent used for M344, flavopiridol and sinapinic acid), corresponding to 0.6 % of total culture volume. Afterwards, the cells were incubated for 72 h in 125 mL-shake flasks. Quantification was performed on a QX200 ddPCR system (Bio-Rad) as described in the examples below. Shown are values ± 95 % CI as calculated by the QX Manager software (Bio-Rad).

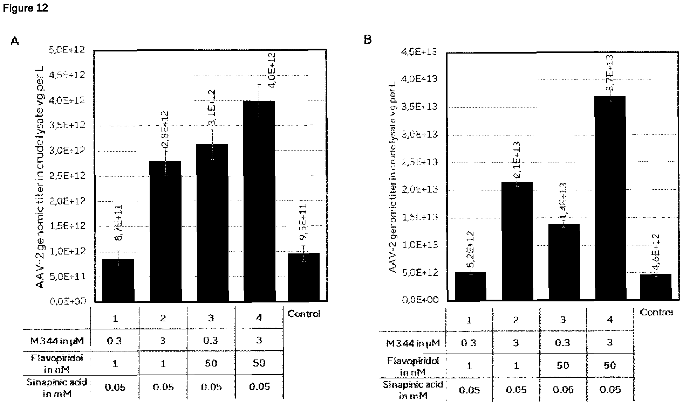

Fig. 12   shows AAV2 genomic titers 72 h after transient transfection for different concentrations of flavopiridol, M344 and sinapinic acid. FreeStyle 293F (see A) or Viral Production (see B) cells were transfected in 125 mL-shake flasks, enhancers were added from separated stock solution 5 h after transient transfection in the indicated concentrations. DMSO control received 180 μL DMSO (solvent used for M344, flavopiridol and sinapinic acid), corresponding to 0.6 % of total culture volume. Quantification was performed on a QX200 ddPCR system (Bio-Rad). Shown are values ± 95 % CI as calculated by the QX Manager software (Bio-Rad).

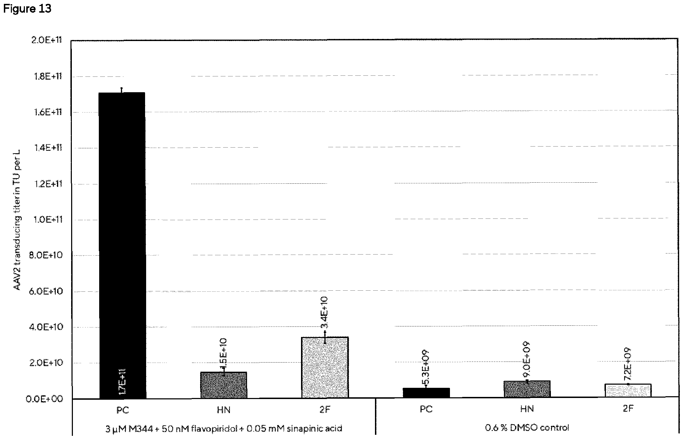

Fig. 13 shows AAV2 transducing titers of harvested cell lysate 72 h after transient transfection in 125 mL-shake flasks. Viral Production Cells 2.0 (PC), the suspension-adapted HEK293 clone (HN) and FreeStyle 293-F cells (2F) were transfected in 125 mL-shake flasks, enhancers were added (3 μM M344, 50 nM flavopiridol, 0.05 mM sinapinic acid) from separated stock solution 5 h after transient transfection in the indicated concentrations. DMSO control received 180 μL DMSO (solvent used for M344, flavopiridol and sinapinic acid), corresponding to 0.6 % of total culture volume. Transduction assay was performed asdescribed in the examples below. Flow cytometry analysis was performed on an iQue® 3 flow cytometer (Sartorius AG). Shown are means ± MAD with n = 2.

## DETAILED DESCRIPTION

The following description serves to deepen the understanding of the present disclosure and shall be understood to complement and be read together with the description of exemplary embodiments of the present disclosure as provided in the above section of this description. It is to be understood that this invention is not limited to the particular embodiments, methodologies, protocols and reagents described herein as these may vary within the scope set by the claims. It is also to be understood that terminology used herein is for the purpose of describing particular embodiments only and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

As explained in the summary of the invention, the different aspects and embodiments of the invention disclosed herein make important contributions to the art by providing improved viral vector production.

### Method according to the first aspect of the invention

According to a first aspect, a viral vector production method is provided, comprising following steps:

(a) providing a mammalian cell configured to produce a viral vector;
(b) contacting the mammalian cell with a flavonoid and/or M344;
(c) culturing the mammalian cell in presence of the flavonoid and/or M344 to produce a viral vector; and
(d) optionally, harvesting the viral vector.

The method according to the present invention advantageously allows for improved viral vector production. By contacting the mammalian cell configured to produce a viral vector with the flavonoid and/or M344 the mammalian cells are impacted such that a higher yield of viral vector and higher quality of viral vector can be produced. As demonstrated in the examples below, the inventors found that both the genomic but also the transducing viral vector titer can be markedly increased by contacting the mammalian cell with a flavonoid and/or M344. As a result, the yield of viral vector that can be applicable for gene therapy is higher and of higher quality.

The individual steps and preferred embodiments of the method according to the first aspect will now be described in detail.

### The viral vector

The viral vector according to the present disclosure is any viral material suitable for delivering genetic material into cells (e.g. alone or in conjunction with further viruses or biochemical cues). In particular, as used herein, the terms "virus vector," "viral vector," and "gene delivery vector" refer to a virus particle that functions as a nucleic acid delivery vehicle, and which comprises a nucleic acid molecule packaged within a viral particle. A viral vector may be suitable for application in gene therapy, i.e. allows for using nucleic acids to repair malfunctioning DNA sequences or to introduce a compensatory change that will restore the normal physiological functions of the cell. Such gene delivery is also referred to as transduction. In general, a viral vector contains one or more target genes, also referred to as "transgenes". The target genes can be transduced into a cell, e.g. patient cells, autologous cells, or allogenic cells, which can happen in vivo or in vitro or ex vivo. The one or more target genes are then either directly available for protein expression in the cell as non-integrative vectors which degrade naturally over time or are integrated into the nuclear DNA of the cell. As used herein, a "transgene" or "target gene" is a nucleic acid that is introduced into the genome, including but not limited to genes or nucleic acid having sequences which are not normally present in the viral vector genes, such as an AAV or LV, which are present but not normally transcribed and translated ("expressed") in the viral vector genome, such as AAV or LV genome, or any other gene or nucleic acid which one desires to position between the viral vector repeat sequences, such as AAV's ITR sequences or the LV's LTR sequences. A transgene may include one or more transcriptional regulatory sequences and any other nucleic acid, such as introns, that may be necessary for optimal expression of a selected nucleic acid. A transgene can be as few as a couple of nucleotides long, but can preferably be at least about 50, 100, 150, 200, 250, 300, 350, 400, or 500 nucleotides (nt) long. A transgene can comprise coding or non-coding sequences.

A number of viruses have been suitable as viral vectors for gene therapy, which are known by the skilled person, see e.g. Roldao et al., 2017, Comprehensive Biotechnology, vol. 1, pp. 633-656. A viral vector may be selected from retroviruses, such as lentivirus, adenoviruses, herpes simplex, vaccinia, and adeno-associated virus (AAV). According to a preferred embodiment, the viral vector is a lentivirus (LV) or an adeno-associated virus (AAV). As demonstrated in the Examples below, the method according to the present disclosure improves the production of AAV and LV, particularly by increasing the genomic and transducing titer (see Figs. 3 to 13).

Herein the term lentivirus is interchangeable used with the terms lentiviruses, LV, LVs, or lentiviral vector, all of which refer to a viral vector which is based on a native lentivirus but which was produced recombinantly. Similarly, the term adeno-associated virus is interchangeable used with the terms recombinant AAV, rAAV, rAAVs, adeno-associated viruses, or AAV vector, all of which refer to a viral vector which is based on a native adeno-associated virus, but which was produced recombinantly. AAV is a small virus capable of infecting humans but not known to cause any disease. AAV can infect both dividing and non-dividing cells but mostly stays as episomal, performing long and stable expression. These features make AAV a very attractive candidate for creating viral vectors for gene therapy. On the other hand, LV have the ability to integrate into the genome of non-dividing cells and allow for permanent gene therapy. The viral genome in the form of RNA is reverse-transcribed when the virus enters the cell to produce DNA, which is then inserted into the genome by the viral integrase enzyme. According to a further embodiment, the viral vector is suitable for gene therapy and preferably selected from the group consisting of retrovirus, such as lentivirus, or adenovirus, or adeno-associated virus (AAV), or hybrids thereof.

As used herein, the term "adeno-associated virus" (AAV), includes but is not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV6), AAV type 7 (e.g, AAV7), AAV type 8 (e.g, AAV8), AAV type 9 (e.g, AAV9), AAV type 10 (e.g, AAV10), AAV type 11 (e.g, AAV11), AAV type 12 (e.g, AAV12), AAV type 13 (e.g, AAV13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

According to a preferred embodiment, the mammalian cell is modified (preferably prior to step (a)) to be configured to produce a viral vector. In other embodiments, it may also be started directly with the mammalian cell without any active modification step by purchasing a mammalian cell that is capable of producing the desired viral vector, e.g. a so-called packaging cell or stable cell which contains all genetic elements required for expressing the viral vector, typically without the target gene(s). In some embodiments, the applied mammalian cell may from the beginning on also be itself capable of expressing one or more genes which are required for producing the viral vector, e.g. HEK293 cells are known to express E1A and E1B which are required for producing AAV.

According to a preferred embodiment, the mammalian cell is modified by transiently transfecting the mammalian cell with one or more plasmids for viral vector production allowing for transient viral vector production (see scheme in Fig. 1). Transiently transfected mammalian cells allow essentially for a single run of viral vector manufacturing, such that for each manufacturing run a transfection has to be performed. According to an alternative preferred embodiment, the mammalian cell is modified by genome editing the mammalian cell with one or more nucleic acid molecules for viral vector production allowing for stable viral vector production (see scheme in Fig. 2). The stable integration allows for using a stock, e.g. cell bank or master cell bank, of modified mammalian cells multiple times without the need to genetically modifying the mammalian cells in advance of each manufacturing run (compared to the transient transfection). This has the advantage that an established process can be repeated multiple times without any necessity to perform transient transfection in advance, simplifying the process and rendering it more efficient. On the other hand, the transient transfection is more flexible, as for each run a different set of genetic elements can be transfected into the cells without complicated genome editing of the mammalian cells in advance. Both ways of modifying the mammalian cells for producing a viral vector are applicable in frame of the method according to the present disclosure and shall not be limiting in any way. Indeed, it is also in scope of the present disclosure to combine transient transfection and stable integration, e.g. by providing some genetic elements stably integrated and other genetic elements via transient transfection. For instance, the transgene gene cassette may be provided by transient transfection, whereas the remaining genetic elements may be stably integrated.

According to a preferred embodiment, the one or more plasmids or the one or more nucleic acid molecules for viral vector production encode at least part of a lentivirus (LV) or at least part of an adeno-associated virus (AAV). In some cases, not all of the virus's genetic elements that are natively present need to be present in the one or more plasmids or the one or more nucleic acid molecules for viral vector production, e.g. if part of the genetic elements is already present in the mammalian cells. For instance, HEK293 cells which are commonly used for producing a viral vector were originally established by transfection of primary human embryonic kidney cells with sheared adenovirus 5 DNA, and it has been shown that HEK293 cells stably express the adenoviral E1A and E1B-55k proteins due to integration of a 4 kbp adenoviral DNA fragment in chromosome 19. Hence, E1A and E1B do not need to be including in the genetic design for modifying such mammalian cell. In addition, for gene therapy, the produced viral vector generally contains one or more genes to be delivered to a patient or another cell. Such one or more genes may be referred to as "target gene(s)". These then typically replace the viral vector production machinery, which is packaged into the plasmid, as it is typically not desired to replicate the viral vector in vivo, i.e. in a potential patient. Therefore, not the native and full viral vector genetic elements may be delivered but only those genetic elements to produce the viral vector including the target gene(s). In some embodiments, the target genes are therapeutic nucleic acids, such as therapeutic DNA or RNA. In other embodiments, the target genes include a reporter gene. In preferred embodiments, the reporter gene can be detected by antibody-based assays. In further preferred embodiments, the reporter gene is a fluorescent molecule. Exemplary fluorescent molecules suitable as reporter gene are GFP, eGFP, mGFP, eYFP, citrine, eCFP, mCFP, Cerulean, dtTomato, and any variants thereof. In some embodiments, the reporter gene is a beta-galactosidase, luciferase or glutathione S-transferase, or any variant thereof. In particular embodiments, the target gene is suitable for screening assays or markers, e.g. fluorescence proteins, such as green fluorescent protein (GFP) or a derivative thereof, which are used for visualizing transduction of the viral vector.

According to a preferred embodiment for producing LV, the one or more plasmids or the one or more nucleic acid molecules for viral vector production encode one or more of the group comprising long terminal repeats (LTRs), polypurine tract (PPT), Rev response element (RRE), psi packaging signal, HIV-1 gag-pol, rev proteins, and enveloped glycoprotein, preferably all of the aforementioned. The encoded genes may be provided on a single or multiple plasmids or a single or multiple nucleic acid molecules. According to one embodiment, more than one plasmid is provided for producing the LV. For instance, two, three, four, five, six, seven, eight, nine or ten plasmids may be provided. In such case, the plasmids encode the same or different genetic elements required for producing the LV. According to a specific, non-limiting embodiment, following plasmid system is used:

- One expression plasmid comprising the target gene, such as a therapeutic nucleic acid (e.g. therapeutic RNA or DNA) or a reporter gene, in particular a fluorescent protein (e.g. GFP or a derivative thereof),
- Three packaging plasmids comprising: (1) a plasmid encoding at least rev proteins, (2) a plasmid encoding at least HIV-1 gag-pol and Rev response element (RRE), and (3) a plasmid encoding enveloped glycoprotein such as VSV-G.

However, any distribution of genetic elements on the one or more (e.g. two, three, four or five) plasmids for producing the LV is possible for use in a method according to the present disclosure. According to a one embodiment of the method according to the first aspect, the viral vector is LV and two, three or four plasmids are transfected into the mammalian cell for producing the LV, wherein the mammalian cell is preferably HEK293.

According to a preferred embodiment for producing AAV, the one or more plasmids or the one or more nucleic acid molecules for viral vector production encode one or more of the group comprising Rep78, Rep68, Rep52, Rep40, VP1, VP2, VP3, ITR, AAP, MAAP, X Gene, VA RNA, E4orf6, and E2A, preferably all of the aforementioned, for producing an AAV. According to another embodiment, the one or more plasmids or the one or more nucleic acid molecules for viral vector production encode one or more of the group comprising Rep78, Rep68, Rep52, Rep40, VP1, VP2, VP3, ITR, AAP, MAAP, X Gene, VA RNA, E4orf6, E1A, E1B, and E2A, preferably all of the aforementioned, for producing an AAV. In any of these embodiments, the mammalian cell may already encode one or more of the genetic elements for producing the AAV. For instance, E1A and E1B may already be produced by the mammalian cell, e.g. HEK293, wherein a plasmid or nucleic acid molecule can support the cellular expression by additional copies of E1A or E1B or the cellular expression may be considered sufficient. The encoded genes may be provided on a single or multiple plasmids or a single or multiple nucleic acid molecules. According to one embodiment, more than one plasmid is provided for producing the AAV. For instance, two, three, four, five, six, seven, eight, nine or ten plasmids may be provided. In such case, the plasmids encode the same or different genetic elements required for producing the AAV. According to a specific, non-limiting embodiment, following plasmid system is used:

- One expression plasmid comprising the target gene, such as a therapeutic nucleic acid (e.g. therapeutic RNA or DNA) or a reporter gene, in particular a fluorescent protein (e.g. GFP or a derivative thereof),
- One packaging plasmids comprising: a plasmid encoding at least Rep78, Rep68, Rep52, Rep40, VP1, VP2, VP3, AAP, MAAP, X Gene, VA RNA, E4orf6, and E2A, optionally also E1A and E1B.

However, any distribution of genetic elements on the one or more (e.g. two, three, four or five) plasmids for producing the AAV is possible for use in a method according to the present disclosure. According to one embodiment, the sequences may also be split, such that only transduction with two or more rAAVs leads to assembled transgenes. According to a one embodiment of the method according to the first aspect, the viral vector is AAV, and two or three plasmids are transfected into the mammalian cell for producing the AAV, wherein the mammalian cell is preferably HEK293.

**Transient transfection**

According to a preferred embodiment, the method according to the present disclosure comprises modifying the mammalian cell, e.g. prior to step (a), by transiently transfecting one or more plasmids into the mammalian cell (see scheme in Fig. 1). These one or more plasmids comprise one or more genetic elements required for producing the viral vector. According to a preferred embodiment, the one or more plasmids comprise all genetic elements required for producing the viral vector of interest, e.g. all genetic elements that are required for producing the AAV or LV.

According to a preferred embodiment, transiently transfecting comprises (x) combining the mammalian cell with the one or more plasmids for viral vector production and a transfection reagent; and (y) incubating the mammalian cell, the one or more plasmids for viral vector production and the transfection reagent to generate a transiently transfected cell. In some embodiments, step (b) according to the method of the present disclosure is performed prior to step (y), such that the flavonoid and/or M344 is/are present during incubating the mammalian cell, the one or more plasmids for viral vector production and the transfection reagent to generate a transiently transfected cell. This may be advantageous to have an early and possibly increased effect of the flavonoid and/or M344 on the mammalian cell. For the step of combining the mammalian cell with the one or more plasmids for viral vector production and a transfection reagent (see step (x)) different procedures may be followed, including:

- combining a cell culture medium with the one or more plasmids for viral vector production and the transfection reagent to form a transfection composition;
- optionally, mixing the transfection composition of (i);
- optionally, incubating the transfection composition of (i) or (ii);
- combining the transfection composition of (i), (ii) or (iii) and the mammalian cell; and
- optionally, the transfection composition of (i), (ii), or (iii) or the composition of (iv) further comprises the flavonoid and/or M344;

or alternatively,

- adding the one or more plasmids for viral vector production and the transfection reagent to the mammalian cell present in a cell culture medium, optionally further comprising the flavonoid and/or M344.

Both methods are suitable for transfecting the mammalian cells with the one or more plasmids for viral vector production. In some embodiments, the flavonoid and/or M344 as disclosed herein is combined with the transfection composition, such that the flavonoid and/or M344 is preferably added at the same time as the one or more plasmids for viral vector production and the transfection reagent to the mammalian cell present in a cell culture medium. In such embodiments, step (b) and step (a) are performed simultaneously, such that afterwards culturing step (c) can be performed. Alternatively, contacting step (b) may be performed in addition to adding the flavonoid and/or M344 to the transfection composition, which may be advantageous to adapt the concentration or content of flavonoid and/or M344.

For the step of incubating the mammalian cell, the one or more plasmids for viral vector production and the transfection reagent to generate a transiently transfected cell according to the prior embodiment (see step (y)), different incubation times are possible. In one embodiment, the incubation step comprises incubating for at least 5 min, at least 10 min, at least 15 min, at least 30 min, at least 45 min, at least 1 hour, at least 2 hours, at least 3 hours, preferably at least 4 hours. It may be advantageous to have longer incubation times such as at least 2 hours, at least 3 hours, at least 4 hours in order to ensure that as much plasmid has been introduced into the mammalian cell as possible. Accordingly in some embodiments, it is incubated for a range of 1 to 48 hours, 2 to 36 hours, 3 to 24 hours, preferably 4 to 12 hours, more preferably 4 to 8 hours or 4 to 6 hours. According to a specific embodiment, it is incubated for 1 to 8 hours. Incubations times of e.g. 1 to 12 hours, preferably 2 to 8 hours or 4 to 6 hours, are advantageous for the method according to the present disclosure to make sure that the one or more plasmids have been introduced into the mammalian cell. However, according to some embodiments, the enhancer may be added before or during transiently transecting the mammalian cell. For instance, during step (x) or during step (y) above contacting step (b) as disclosed herein may be performed, such that the mammalian cell is contacted with the one or more plasmids for viral vector production and a transfection reagent and the enhancer, e.g. flavonoid and/or M344. Thus step (x) and (b) may be performed simultaneously. As a result, incubation would take place in presence of the enhancer. Afterwards, the mammalian cell may be further contacted with the enhancer, i.e. step (b) is performed, or the mammalian cell may be directly cultured according to step (c).

According to a preferred embodiment, transiently transfecting the mammalian cell comprises transfecting with at least 0.1 µg plasmid DNA per 1 million cells, preferably at least 0.5 µg or at least 1 µg plasmid DNA per 1 million cells. These amounts have been proven suitable for transient transfection, as is demonstrated in the Examples below.

According to a preferred embodiment, the transfection reagent is polymer-based or lipid-based, preferably being cationic, such as polyethylene imine or a derivative thereof. In a particular example (also shown below), the transfection reagent may comprise polyethyleneimine. However, the skilled person is well-aware of different transfection reagents and will readily understand that different transfection reagents are applicable in frame of the method according to the present disclosure.

**Stable integration**

The mammalian cell may be modified by genome editing the mammalian cell with one or more nucleic acid molecules for viral vector production allowing for stable viral vector production (see scheme in Fig. 2). The stable integration is advantageous in that it allows using the same stock of genome edited mammalian cells multiple times without the need to genetically modifying the mammalian cells in advance of culture. According to a preferred embodiment, genome editing the mammalian cell comprises stably integrating the one or more nucleic acid molecules into the chromosome of the mammalian cell to be capable of stably producing a viral vector. The integration can be performed by providing at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same. In addition, the genetic elements required for producing the viral vector may be provided in form of a template to be integrated into the genome which may be cut by the at least one site-directed endonuclease, e.g. a CRISPR-nuclease. The way of integrating the genetic elements into the genome of the mammalian cell shall not be limited and different techniques are available to the skilled person. According to one particular embodiment, however, the one or more genetic elements required for viral vector production may be integrated using a CRISPR-nuclease, e.g. Cas12a or Cas9.

According to a preferred embodiment of modifying the mammalian cell by genome editing with one or more nucleic acid molecules for viral vector production allowing for stable viral vector production, the one or more nucleic acid molecules comprise an induction system, such as in particular one or more inducible promotors. Thereby, the expression of viral vector genes can be regulated. This has the advantage that the viral vector genes are not uncontrollably expressed, which can have a negative impact on the mammalian cell viability, as is e.g. known for some Rep proteins of AAV. It is preferred that one or more inducible promotors are used which can be induced by the same or different triggers. For instance, it may be desired to induce all promotors at the same time in order to start expression of the viral vector genes stably integrated into the genome of the mammalian call. Alternatively, it may be desired to orchestrate the expression of the viral vector genes stably integrated into the genome of the mammalian cell, e.g. by providing sequentially different triggers for inducing the promotor to start protein expression. The type and sequence of inducible system shall not be limiting in frame of the present disclosure and the skilled person is well-aware of different suitable induction systems.

**Step (a)**

Step (a) of the method according to the first aspect comprises providing a mammalian cell configured to produce a viral vector. The mammalian cell can be provided in any manner known by the person skilled in the art. For instance, the mammalian cell can be provided in form of a cryopreserved cell bank which is redispersed in suitable cell culture medium. Alternatively, the mammalian cell can undergo a scale up or seed train process, wherein the mammalian cell is cultured in cell culture medium to a desired cell number followed by transfer into a larger cell culture vessel or dish or bioreactor and the dilution with further cell culture medium. The mammalian cell can also be provided as a mixture comprising the mammalian cells and a cell culture medium suitable for culturing such mammalian cell. In a particular embodiment, the mammalian cell is a HEK293 cell or a derivative thereof and is provided in form of a suspension with cell culture medium suitable for culturing the HEK293 cell or derivative thereof.

As disclosed above different methods are suitable for modifying the mammalian cell to be configured to produce a viral vector, such as transient transfection of one or more plasmids or genome editing with one or more nucleic acids, wherein preferably the one or more plasmids or the one or more nucleic acids comprise the genetic element required for producing the viral vector in the mammalian cell. For more details on how to modify the mammalian cell to be configured to produce the viral vector, it is referred to the above sections.

According to a preferred embodiment, step (a) further comprises culturing the mammalian cell configured to produce a viral vector. This may be desirable in order to generate a sufficient number of mammalian cells. According to one embodiment, step (a) comprises culturing the mammalian cell to generate at least 1 million viable cells per mL, preferably at least 2 million viable cells per mL, more preferably at least 3 million cells per mL. The number of cells to be generated in culture depends on the specific needs, i.e. if a high number is desired, also higher cell numbers can be obtained by culture, e.g. at least 5 million cells per mL, at least 10 million cells per mL, at least 15 million cells per mL, at least 20 million cells per mL, or at least 25 million cells per mL. If a low number of cells is desired also lower numbers are possible, e.g. at least 0.1 million cells per mL, at least 0.25 million cells per mL, or at least 0.5 million cells per mL.

After culturing, the mammalian cell may be diluted throughout the step of modifying the mammalian cell such that it is configured to produce a viral vector. For instance, the mammalian cell may be diluted by transiently transfecting the mammalian cell, such that it is configured to produce a viral vector, e.g. AAV or LV.

According to a preferred embodiment, step (a) of the method according to the first aspect comprises providing a mammalian cell configured to produce a viral vector, wherein the mammalian cell has a number of at least 0.5 million cells per mL, at least 0.75 million cells per mL, or preferably at least 1 million cells per mL. Such concentrations are desired for subsequent contacting step (b). Such concentrations have been found advantageous for producing a viral vector, such as AAV or LV, as is also shown in the examples below. According to a preferred embodiment, the mammalian cell has a number of 0.5 to 10 million cells per mL, 0.75 to 7.5 million cells per mL, or preferably at least 1 to 5 million cells per mL.

In case step (a) further comprises culturing the mammalian cell configured to produce a viral vector, such culture may be performed in any mode suitable and known by the person skilled in the art. Typical culturing processes of a mammalian cell encompass batch, fed-batch, and perfusion culture or a combination of any of the aforementioned. For instance, the mammalian cell may first be cultured in batch mode, e.g. until a desired cell number is generated, followed by switch into fed-batch culture or perfusion culture. According to one embodiment, step (a) further comprises culturing the mammalian cell, e.g. HEK293 cell, in batch culture to generate at least 1 million viable cells per mL, preferably at least 2 million viable cells per mL, more preferably at least 3 million cells per mL.

According to a preferred embodiment, step (a) further comprises culturing the mammalian cell in suspension culture. Culturing in suspension culture has the advantage that typically higher cell numbers can be achieved than for adherent cells. Also, addition of microcarrier systems can be avoided, which may be required for adherent cells. It is also within the scope of the present disclosure that the mammalian cell is originally adherent and then adapted for suspension culture. For instance, according to one embodiment, prior to step (a), an adherent mammalian cell is suspension adapted, optionally followed by modifying the mammalian cell such that the mammalian cells are configured to produce the viral vector (e.g. by transient transfection or genome editing), followed by providing the mammalian cell configured to produce the viral vector according to step (a). Alternatively, it may also be directly started with mammalian cell suitable for suspension culture to avoid the step of suspension-adaption. Different types of mammalian cells, including suspension and adherent mammalian cell, are available to the person skilled in the art (see e.g. Roldao et al., 2017, Comprehensive Biotechnology, vol. 1, pp. 633-656).

According to one embodiment, step (a) comprises culturing the mammalian cell in suspension culture to generate at least 1 million viable cells per mL, preferably at least 2 million viable cells per mL, more preferably at least 3 million cells per mL. In such embodiment, the mammalian cell may be a HEK293 cell, and it is configured to produce an AAV or LV.

**Mammalian cell**

In the method according to the present disclosure, a mammalian cell is provided. A mammalian cell is a cell that is of mammalian origin. However, the cell does not need to be identical to a cell obtained in a mammalian but can be modified, engineered or naturally/artificially changed. For instance, the mammalian cell may be a cell with mammalian origin, however, may contain one or more genetic changes to propagate it repeatedly and possibly also infinitely. The term "mammalian cell" may be interchangeable used with "mammalian cells" and shall cover both the singular and plural form. The mammalian cell is preferably a mammalian cell line. According to a preferred embodiment, the mammalian cell is a human-derived cell line, which advantageously has the ability to provide the suitable post-translational modifications for therapy in humans. This means that the produced viral vector has similar post-translational modification, as if the native virus would have infected a human cell resulting in virus production.

The mammalian cell can be selected from the group consisting of HeLa, Human embryonic kidney 293 (HEK293), BSC-1, SW480, Baby hamster kidney (BHK), BHK-21, Vero E6, U2OS, A549, HT1080, CAD, P19, NIH 3T3, L929, N2a, Chinese hamster ovary (CHO), MCF-7, Y79, SO-Rb50, Hep G2, DUKX-X11, J558L, HuH-7, MDCK, or HepG2 cells or derivatives thereof, e.g. sub-cell lines such as for HEK293 also HEK293-T, HEK293-F, HEK293-FT, etc. According to a preferred embodiment, the mammalian cell is selected from the group consisting of HEK293, A549, BSC-1, SW480, Baby hamster kidney (BHK), Vero E6 and MDCK cells or a derivative thereof. According to an even more preferred embodiment, the mammalian cell is selected from a HEK293 cell or a derivative of a HEK293 cell. HEK293 is particularly advantageous as it is well-characterized, widely used and very susceptible to transfection. The HEK293 cell was established by transforming human embryonic kidney cells with sheared adenovirus type 5 DNA. Accordingly, the mammalian cell may be a HEK293 cell or a derivative of a HEK293 cell, e.g. selected from HEK293, HEK293-T, HEK293-F, HEK293-FT, HEK293-E and HEK293-6E. A derivative of a HEK293 cell may also encompass a HEK293 cell that is adapted to suspension culture and/or adapted to a particular type of cell culture medium.

According to a preferred embodiment, the HEK293 cell or the derivative of a HEK293 cell has been adapted for suspension culture. This is particularly advantageous, as it allows for obtaining higher cell numbers compared to adhesive HEK293 cells. It may also be that originally adhesive HEK293 cells are used which are subsequently suspension adapted (prior to step (a)) and then modified such that it is configured to produce a viral vector (e.g. by transient transfection or genome editing).

**Step (b)**

Step (b) of the method according to the first aspect of the present disclosure comprises contacting the mammalian cell with a flavonoid and/or M344. Contacting may comprise adding the flavonoid and/or M344 in solid form or in dissolved form to the mammalian cell. Preferably, the mammalian cell, e.g. HEK293 cell or derivative thereof, is provided as a suspension with cell culture medium in step (a) and then the mammalian cells are contacted with the flavonoid and/or M344 by adding the flavonoid and/or M344 to the mammalian cell or adding the mammalian cell to the flavonoid and/or M344.

Apart from the flavonoid and/or M344, the mammalian cell may also be contacted with further compounds. Such compounds can be useful for the culture of the mammalian cell, e.g. cell culture medium, or may be required for contacting the mammalian cell with the flavonoid and/or M344. For instance, the flavonoid and/or M344 may be stabilized or dissolved with one or more other compounds with which the mammalian cells are also contacted in step (b). As cell culture medium any composition suitable for culturing the mammalian cell is applicable. Such cell culture media are well-known to the skilled person and shall not limit the scope of the present disclosure. Specific examples includes the cell culture media used in the Examples below, see e.g. HEK ViP NB and HEK TF (Sartorius Xell GmbH), and DMEM (Pan-Biotech).

According to a preferred embodiment, the flavonoid and/or M344 is/are dissolved prior to contacting step (b). Such embodiment is advantageous, as it allows to precisely adjust the added amount of the flavonoid and/or M344, which can be challenging by using flavonoid and/or M344 in solid form. Nevertheless, is may also be possible within the scope of the present disclosure to add the flavonoid and/or M344 in solid form in the desired amount. In case the flavonoid and/or M344 is/are dissolved, it is preferred to dissolve the flavonoid and/or M344 in a solvent which allows for dissolving the flavonoid and/or M344 in an amount desired for contacting step (b). Hence, the solvent may be any solvent in which flavonoid and/or M344 can be dissolved sufficiently. According to a preferred embodiment, the flavonoid and/or M344 is/are dissolved in an organic solvent, preferably dimethyl sulfoxide (DMSO), ethanol, dimethyl formamide (DMF), or chloroform, more preferably DMSO. DMSO was also used in the Examples below allowing for dissolving a sufficient amount of flavonoid and/or M344 but also having neglectable side effects on the mammalian cell. However, other solvents are readily available to the skilled person. For instance, flavonoid and/or M344 may be directly dissolved in cell culture medium. In such embodiment, the flavonoid and/or M344 containing cell culture medium may be added directly to the mammalian cells, which are provided e.g. in form of centrifuged cells or in form of a suspension with cell culture medium. In any case, the flavonoid and/or M344 in cell culture medium shall be added such that the desired amount of flavonoid and/or M344 is present in presence with the mammalian cell.

Contacting the mammalian cell in step (b) preferably results in a concentration of the flavonoid and/or M344 in a composition with the mammalian cell that increases the genomic and/or transducing viral vector titer. In one embodiment, the concentration of the flavonoid and/or M344 in a composition with the mammalian cell results in an increase of the genomic viral vector titer by at least 10%, preferably at least 25%, compared to a viral vector production method without contacting the mammalian cell with the flavonoid and/or the M344. In one embodiment, the concentration of the flavonoid and/or M344 in a composition with the mammalian cell results in an increase of the transducing viral vector titer by at least 10%, preferably at least 25%, compared to a viral vector production method without contacting the mammalian cell with the flavonoid and/or the M344. In one embodiment, the concentration of the flavonoid and/or M344 in a composition with the mammalian cell results in an increase of the genomic viral vector titer and the transducing viral vector titer by at least 10%, preferably at least 25%, compared to a viral vector production method without contacting the mammalian cell with the flavonoid and/or the M344. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

According to a preferred embodiment, step (b) comprises contacting the mammalian cell with the flavonoid resulting in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell. According to a further preferred embodiment, step (b) comprises contacting the mammalian cell with the flavonoid resulting in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell. As demonstrated in the Examples below, such concentrations and concentration ranges lead to advantageously improved viral vector production. Specifically, such concentrations increase the genomic titer and/or increase the transducing titer in the disclosed viral vector production method (see Figs. 3, 4, 5, 11, 12A, 12B, and 13). The composition may comprise the mammalian cell, the flavonoid and a cell culture medium. The composition may further comprise the solvent in which the flavonoid is dissolved, e.g. an organic solvent, such as DMSO. The composition may comprise further compounds suitable for culturing the mammalian cell and/or producing the viral vector.

According to a preferred embodiment, step (b) comprises contacting the mammalian cell with the M344 resulting in a concentration of at least 0.5 $\mu$M, at least 1 $\mu$M, at least 1.5 $\mu$M, at least 2 $\mu$M, at least 2.5 $\mu$M, at least 3 $\mu$M, at least 4 $\mu$M, at least 5 $\mu$M, at least 6 $\mu$M, at least 7 $\mu$M, at least 8 $\mu$M, at least 9 $\mu$M, at least 10 $\mu$M, preferably at least 5 $\mu$M in a composition with the mammalian cell. As demonstrated in the Examples below, such concentrations lead to advantageously improved viral vector production. Specifically, such concentrations increase the genomic titer and/or increase the transducing titer in the disclosed viral vector production method (see Figs. 6, 7, 8, 9, 10, 11, 12A, 12B, and 13). The composition may comprise the mammalian cell, the M344 and a cell culture medium. The composition may further comprise the solvent in which the M344 is dissolved, e.g. an organic solvent, such as DMSO. The composition may comprise further compounds suitable for culturing the mammalian cell and/or producing the viral vector.

After contacting step (b) of the method according to the first aspect of the present disclosure, the flavonoid and/or M344 preferably remains in the composition with the mammalian cell or may be removed after incubation. Alternatively, the flavonoid and/or M344 may be washed out or continuously added throughout viral vector production. In one embodiment, the flavonoid and/or M344 are contacted with the mammalian cell, followed by culture, wherein the flavonoid and/or M344 essentially remains in the composition throughout culture, wherein preferably the culture comprises a batch culture. In another embodiment, the flavonoid and/or M344 are contacted with the mammalian cell, followed by culture, wherein further flavonoid and/or M344 are added throughout the culture, wherein preferably the culture comprises a fed-batch culture or a perfusion culture. In another embodiment, the flavonoid and/or M344 are contacted with the mammalian cell, followed by culture, wherein further flavonoid and/or M344 are diluted throughout the culture, wherein preferably the culture comprises a fed-batch culture or a perfusion culture.

According to one embodiment, step (b) comprises contacting the mammalian cell with a flavonoid. In such embodiment, no M344 is added. Contacting the mammalian cell with a flavonoid, such as flavopiridol or a derivative thereof, is highly advantageous, as it improves the viral vector production, as is shown in the Examples below (see Figs. 3, 4, and 5).

According to another embodiment, step (b) comprises contacting the mammalian cell with a M344. In such embodiment, no flavonoid is added. Contacting the mammalian cell with M344, especially at elevated concentrations, e.g. at least 5 $\mu$M in a composition with the mammalian cell, is highly advantageous, as it improved the viral vector production, as is shown in the Examples below (see Figs. 6, 7, 8, 9, and 10).

According to one embodiment, step (b) comprises contacting the mammalian cell with the flavonoid, preferably flavopiridol, and the M344. Contacting the mammalian cell with a flavonoid, such as flavopiridol or a derivative thereof, and M344, especially at elevated concentrations, e.g. at least 0.5 $\mu$M, at least 2 $\mu$M, or at least 5 $\mu$M in a composition with the mammalian cell, is highly advantageous, as it improved the viral vector production, as is shown in the Examples below (see Figs. 11, 12A, 12B, and 13), wherein in some cases synergistic effects have been observed. In such an embodiment, the mammalian cell may be a human-derived cell line, such as a HEK293 cell or a derivative thereof. Furthermore, the viral vector may be a AAV or LV.

According to a preferred embodiment, the genomic viral vector titer and/or transducing viral vector titer is increased compared to a viral vector production method without contacting the mammalian cell with the flavonoid and/or the M344. By increasing the viral and/or transducing titer the resulting produced viral vector can be produced more efficiently and with higher yield and/or has a higher quality. Such increase can be obtained by using the flavonoid, e.g. flavopiridol or a derivative thereof, alone. Such increase can also be obtained by using the M344, e.g. at elevate concentrations, such as at least 5 $\mu$M in the composition with the mammalian cell, alone. Finally, such increase, which is in some cases synergistic can also be obtained by the combination the flavonoid and the M344.

According to one embodiment, the flavonoid and/or M344 advantageously increases the genomic and/or transducing viral vector titer. According to a preferred embodiment, the genomic viral vector titer is increased by at least 10%, preferably at least 25%, compared to a viral vector production method without contacting the mammalian cell with the flavonoid and/or the M344. According to a preferred embodiment, the transducing viral vector titer is increased by at least 10%, preferably at least 25%, compared to a viral vector production method without contacting the mammalian cell with the flavonoid and/or M344. According to a further embodiment the genomic viral vector titer and the transducing viral vector titer is increased by at least 10%, preferably at least 25%, compared to a viral vector production method without contacting the mammalian cell with the flavonoid and/or the M344.

Methods of measuring the genomic and transducing viral vector titer are available in the art and known by the person skilled in the art. For instance, the genomic titer may be measured by digital PCR, e.g. droplet digital PCR, AUC, TEM, HCP, or ELISA. Exemplary methods for the detection of the capsid titer of an AAV and methods for the determination of the ratio of full and empty capsids of an AAV are described in WO 2023/139224 incorporated by reference in its entirety. In a particular embodiment, the genomic viral vector titer is measured by droplet digital PCR. The transducing titer which may also be referred to as the infectious titer or infection titer may be measured by a transduction assay. Such transduction assay typically is based on infecting pre-cultured cells using the viral vector and then measuring the infection, e.g. by determining the amount of infected cells, such as by measuring the amount of produced transduced cells. In a particular example (also shown below), the produced viral vector contains a gene encoding GFP, such that after transduction cells produce GFP. GFP-expressing cells can then be easily determined, such as by flow cytometry (e.g. iQue® 3) or microscopically (e.g. by Incucyte).

**Flavonoid**

According to the method, step (b) may comprise adding a flavonoid. Flavonoids, a group of natural substances with variable phenolic structures, are well-known for their beneficial effects on health. Flavonoids are now considered as an indispensable component in a variety of nutraceutical, pharmaceutical, medicinal and cosmetic applications. This is attributed to their anti-oxidative, antiinflammatory, anti-mutagenic and anti-carcinogenic properties coupled with their capacity to modulate key cellular enzyme function (see Panche et al., 2016, Journal of Nutritional Science, Vol. 5, e47, pp. 1-15).

According to a preferred embodiment, the flavonoid is chemically synthesized, preferably a chemically synthesized flavone. Chemically synthesized flavonoids have the advantage that they are well defined in their composition such that they are applicable to chemically defined cell culture media.

According to a preferred embodiment, the flavonoid is selected from the group consisting of isoflavones, neoflavonoids, flavones, flavonols, flavanones, flavanonols, flavanols, catechins, anthocyanins and chalcones, preferably the flavonoid is a flavone. According to a preferred embodiment, the flavonoid is selected from one or more of the group consisting of flavopiridol, catechin, genistein, quercetin, wogonin, kaempferol, baicalein, baicalin, fisetin, apigenin, luteolin, chrysin, rohitukine, P-276-00, and any derivative of the aforementioned. Also, further flavonoids or derivatives disclosed in Jain et al., 2012, Mini-Reviews in Medicinal Chemistry, 2012, 12, pp. 1-18 may be used as flavonoid in frame of the present disclosure. According to a preferred embodiment, the flavonoid is flavopiridol or a derivative thereof. As demonstrated in the Examples below, flavonoids, such as flavopiridol or derivatives thereof, improve the viral vector production, particularly by increasing the genomic and/or transducing viral vector titer.

According to a preferred embodiment, the flavonoid inhibits one or more cyclin-dependent kinases (CdK), preferably selected from Cdk1, Cdk2, Cdk3, Cdk4, Cdk5, Cdk6, Cdk7, Cdk8, Cdk9, Cdk10, Cdk11, or a combination thereof. Flavones are known for their ability to inhibit important cell signalling enzymes such as protein kinase C, cyclin-dependent kinases (CdK) and tyrosine kinases. Cyclin-dependent kinases (CdKs) have been recognized as key regulators of cell cycle progression (see Jain et al., 2012, Mini-Reviews in Medicinal Chemistry, 2012, 12, pp. 1-18). The present inventors hypothesize that using a flavonoid, such as flavopiridol or a derivative thereof, for viral vector production inhibits one or more CdKs of the mammalian cell, which has an impact on the cell cycle progression, positively affecting also viral vector production. Without being bound to theory, the flavonoid, such as flavopiridol could indirectly support viral DNA replication and lead to a higher transcription rate and higher packaging efficiency. It is believed that this could be especially rely on the inhibition of CdK9. Accordingly in a preferred embodiment, the flavonoid inhibits at least Cdk9.

According to one embodiment, step (b) comprises contacting the mammalian cell with the flavonoid resulting in a concentration of at least 40 nM in the composition with the mammalian cell, and wherein the flavonoid inhibits at least CdK9. Such concentration and the inhibition of CdK9 was identified as advantageous, resulting in improved viral vector production, as demonstrated in the Examples below (see Figs. 3, 4, 5, 6, 7, 8). In such embodiment, the flavonoid may be flavopiridol or a derivative thereof.

According to one embodiment, step (b) comprises contacting the mammalian cell with the flavonoid resulting in a concentration of at least 40 nM in the composition with the mammalian cell, and wherein the flavonoid is flavopiridol or a derivative thereof. Such concentration of flavopiridol was identified as advantageous, resulting in improved viral vector production, as demonstrated in the Examples below (see Figs. 3, 4, 5, 6, 7, 8).

**M344**

Step (b) may comprise contacting the mammalian cell with a M344. M344, which is also referred to as 4-dimethylamino-N-(6-hydroxycarbamoylhexyl)-benzamide is an inhibitor of histone deacetylase (HDAC). HDACs remove acetyl moieties from lysine residues located on histones and cause changes in gene expression. This has been shown to reduce cancer growth (see Knoche et al., 2022, pLoS ONE 17(9): e0273518). HDAC inhibitors such as sodium butyrate and sodium valproate have been shown in the past to influence rAAV production, at high concentrations in the millimolar range (see WO 2020/033842 A1).

The inventors surprisingly found that the HDAC inhibitor M344 is very potent in improving the viral vector production, as is demonstrated in the examples below, especially for LV and AAV. It was shown that M344 improves the genomic and transducing viral vector titer.

According to a further embodiment, step (b) comprises contacting the mammalian cell with the M344 resulting in a concentration of at least $0.5\,\mu M$, at least $1\,\mu M$, at least $1.5\,\mu M$, at least $2\,\mu M$, at least $2.5\,\mu M$, at least $3\,\mu M$, at least $4\,\mu M$, at least $5\,\mu M$, at least $6\,\mu M$, at least $7\,\mu M$, at least $8\,\mu M$, at least $9\,\mu M$, at least $10\,\mu M$, preferably at least $5\,\mu M$ in a composition with the mammalian cell. According to a preferred embodiment, step (b) comprises contacting the mammalian cell with the M344 resulting in a concentration of $0.5\,\mu M$ to $1000\,\mu M$, $1\,\mu M$ to $900\,\mu M$, $1.5\,\mu M$ to $800\,\mu M$, $2\,\mu M$ to $700\,\mu M$, $2.5\,\mu M$ to $600\,\mu M$, $3\,\mu M$ to $500\,\mu M$, $4\,\mu M$ to $450\,\mu M$, $5\,\mu M$ to $400\,\mu M$, $6\,\mu M$ to $350\,\mu M$, $7\,\mu M$ to $300\,\mu M$, $8\,\mu M$ to $250\,\mu M$, $9\,\mu M$ to $200\,\mu M$, $10\,\mu M$ to $100\,\mu M$, preferably $5\,\mu M$ to $100\,\mu M$ in a composition with the mammalian cell. As demonstrated in the Examples below, such concentrations lead to advantageously improved viral vector production. Specifically, such concentrations increase the genomic titer and/or increase the transducing titer in the disclosed viral vector production method (see Figs. 6, 7, 8, 9, 10, 11, 12A, 12B, and 13).

Contacting the mammalian cell with M344, especially at elevated concentrations, e.g. at least $5\,\mu M$ in a composition with the mammalian cell, is highly advantageous, as it improved the viral vector production, as is shown in the Examples below (see Figs. 6, 7, 8, 9, and 10).

According to one embodiment, step (b) comprises contacting the mammalian cell with the flavonoid, preferably flavopiridol, and the M344. Contacting the mammalian cell with a flavonoid, such as flavopiridol or a derivative thereof, and M344, especially at elevated concentrations, e.g. at least $0.5\,\mu M$, at least $2\,\mu M$, or at least $5\,\mu M$, e.g. $5\,\mu M$ to $100\,\mu M$, in a composition with the mammalian cell, is highly advantageous, as it improved the viral vector production, as is shown in the Examples below (see Figs. 11, 12A, 12B, and 13), wherein in some cases synergistic effects have been observed. In such an embodiment, the mammalian cell may be a human-derived cell line, such as a HEK293 cell or a derivative thereof. Furthermore, the viral vector may be a AAV or LV.

**Step (c)**

Step (c) of the method according to the present disclosure comprises culturing the mammalian cell in presence of the flavonoid and/or M344 to produce a viral vector. Culturing may be understood as cultivating the mammalian cell in an environment suitable for producing the viral vector, such as LV or AAV. Such culture can take place in any format or scale, e.g. in a bioreactor, shake flask, well plate, etc. As is demonstrated in the Examples, the method according to the present disclosure is independent of scale and thus applicable for any type of cultivation method.

Culturing according to step (c) may comprise performing any mode suitable for producing the viral vector. For instance, culturing may comprise performing a batch, fed-batch, or perfusion culture, or any combination thereof. For instance, it may be desirable to produce the viral vector in batch or fed-batch mode when using a mammalian cell that is configured to produce a viral vector by transient transfection. In another example, it may be desirable to produce the viral vector in fed-batch or perfusion culture when using a mammalian cell that is configured to produce a viral vector by genome editing (referred to as stable cell line). Also, combinations of culture modes are within the scope of the present disclosure, e.g. by first culturing in batch mode, followed by perfusion or fed-batch culture.

According to a preferred embodiment, step (c) comprises culturing the mammalian cell for at least 12 hours, at least 18 hours, at least 24 hours, at least 36 hours, preferably at least 48 hours, more preferably at least 72 hours. Culturing the mammalian cell for such time advantageously leads to increased viral vector production. Indeed, longer cultivation duration leads to higher numbers of viral vectors. In the examples below a cultivation duration of about 72 hours was performed showing superior viral vector yields and quality, however, the present disclosure shall not be limited to such specific cultivation duration.

According to a preferred embodiment, step (c) comprises culturing the mammalian cell in suspension culture. Suspension culture is particularly advantageous, as it allows for easy scale up and culture compared to adhesion culture.

According to a particular embodiment, the mammalian cell which are cultured in step (c) are HEK293 cells or a derivative thereof, which are cultured as suspension culture. In such embodiment, the viral vector may be LV or AAV.

**Step (d)**

The method according to the present disclosure optionally further comprises step (d) harvesting the viral vector. In a preferred embodiment, the method comprises step (d), i.e. such step is not optional. Harvesting is typically understood as obtaining the viral vector produced by the mammalian cell after or throughout culture. Hence, harvesting can take place at the end of the production, e.g. by stopping the culture and obtaining the viral vector. Alternatively, harvesting can be performed throughout culture by intermittently or continuously obtaining the viral vector, e.g. by obtaining the cell suspension or a permeate which could be obtained in perfusion culture.

Harvesting of the viral vector can encompass obtaining the viral vector from the mammalian cell (intracellular viral vector) and/or the surrounding cell culture medium, also referred to as supernatant (extracellular viral vector). Some viruses, e.g. AAV8, secrete directly into the medium, such that the viral vector can be directly obtained from the cell culture medium or supernatant (see e.g. WO 2007/127264 A2). Other viral vectors are mostly present within the mammalian cell, such that the cells need to be disrupted or lysed in order to obtain the viral vector. It may be advantageous to use both the supernatant and the cells in order to harvest the viral vector to obtain a high yield of viral vector.

According to one embodiment, step (d) comprises harvesting the viral vector by chemically lysing the mammalian cell after step (c). For this purpose, the mammalian cell may be separated from the cell culture medium or provided together with the cell culture medium. For chemical lysis, the mammalian cell is contacted with a chemical lysis reagent or composition. In particular, a detergent-based chemical lysis reagent or composition may be used for lysing the mammalian cell. Such detergent-based lysis composition is advantageously used in the examples, demonstrating efficient lysis and thus harvesting of the viral vector. However, other methods of disrupting the mammalian cell may be used, e.g. sonication and high-pressure homogenization, physical methods like freeze-thaw, and chemical methods, other mechanical lysis methods. The skilled person is well-aware of different methods of disrupting a cell in order to harvest the viral vector.

According to one embodiment, harvesting step (d) comprises lysing the transiently transfected cells. As is demonstrated in the Examples below this leads to high yields and high quality of the viral vector such as AAV and LV.

**Further embodiments**

The method according to the present disclosure may further comprises step (e) purifying the harvested viral vector. After harvesting, it may be required to purify the viral vector for subsequent steps, e.g. analytics, formulation, etc. Purifying the harvested viral vector may be performed by any method applicable and known by the skilled person. Indeed, various protocols and methods are available in the art for purifying a viral vector, typically including multiple-step processes (also referred to as downstream processing). For example, the harvested viral vector may undergo filtration, centrifugation, chromatography, dialysis, etc. Chromatographic purification may encompass anionic, cationic and/or affinity chromatography.

The method according to the present disclosure may further comprises step (f) formulating the viral vector for gene therapy. Such step may advantageously be performed after the purification of the viral vector. Formulating the viral vector is particularly useful when applying the viral vector for gene therapy but also for storing the viral vector.

According to a preferred embodiment, the steps of the method according to the present disclosure are performed sequentially, i.e. step (a), followed by step (b), followed by step (c), optionally followed by step (d), optionally followed by step (e). In some cases, also step (f) is performed. Some steps may also be performed simultaneously, e.g. the mammalian cell may be provided and contacted with the flavonoid and/or M344 at the same time. Also, as disclosed herein step (b) may be performed during transiently transfecting the mammalian cell. It is referred to the respective sections for the sake of conciseness. Possibly, also culturing step (c) is performed at the same time. In other embodiments, contacting step (b) and culturing step (c) are performed simultaneously. Steps in between the steps may also be included in the method according to the present disclosure, e.g. the method may comprise a further culturing step in advance of step (a) and the step (b).

According to a particular embodiment, the method according to the present disclosure is characterized by the following features:

- the viral vector is AAV or LV;
- the mammalian cell is configured to produce AAV or LV, preferably wherein the mammalian cell is modified by transiently transfecting the mammalian cell with one or more plasmids for AAV or LV production allowing for transient AAV or LV production;
- the mammalian cell is a human-derived cell line, preferably HEK293 or a derivative thereof;
- step (b) comprises contacting the mammalian cell with a flavonoid, preferably flavopiridol or a derivative thereof; and
- the method comprises step (d).

According to a particular embodiment, the method according to the present disclosure is characterized by the following features:

- the mammalian cell is configured to produce a viral vector, preferably wherein the mammalian cell is modified by transiently transfecting the mammalian cell with one or more plasmids for viral vector production allowing for transient viral vector production; and
- step (b) comprises contacting the mammalian cell with the flavonoid, preferably the flavonoid inhibits at least Cdk9, resulting in a concentration of at least 40 nM in a composition with the mammalian cell.

According to a particular embodiment, the method according to the present disclosure is characterized by the following features:

- step (a) comprises providing a mammalian cell configured to produce a viral vector, wherein the mammalian cell has a number of at least 1 million cells per mL; and
- step (b) comprises contacting the mammalian cell with the flavonoid, preferably the flavonoid inhibits at least Cdk9, resulting in a concentration of at least 40 nM in a composition with the mammalian cell.

According to a particular embodiment, the method according to the present disclosure is characterized by the following features:

- the mammalian cell is configured to produce a viral vector, preferably wherein the mammalian cell is modified by transiently transfecting the mammalian cell with one or more plasmids for viral vector production allowing for transient viral vector production; and
- step (b) comprises contacting the mammalian cell with the flavonoid, preferably the flavonoid inhibits at least Cdk9, resulting in a concentration of 1 nM to 1000 nM, preferably 40 nM to 500 nM, in a composition with the mammalian cell

According to a particular embodiment, the method according to the present disclosure is characterized by the following features:

- step (a) comprises providing a mammalian cell configured to produce a viral vector, wherein the mammalian cell has a number of at least 1 million cells per mL; and
- step (b) comprises contacting the mammalian cell with the flavonoid, preferably the flavonoid inhibits at least Cdk9, resulting in a concentration of 1 nM to 1000 nM, preferably 40 nM to 500 nM, in a composition with the mammalian cell

According to a particular embodiment, the method according to the present disclosure is characterized by the following features:

- the mammalian cell is a human-derived cell line, preferably HEK293 or a derivative thereof;
- step (b) comprises contacting the mammalian cell with a flavonoid, preferably flavopiridol or a derivative thereof, resulting in a concentration of the flavonoid in a composition with the mammalian cell that increases the genomic and/or transducing viral vector titer; and
- the method comprises step (d).

According to a particular embodiment, the method according to the present disclosure is characterized by the following features:

- the mammalian cell is a human-derived cell line, preferably HEK293 or a derivative thereof;
- step (b) comprises contacting the mammalian cell with a flavonoid, preferably flavopiridol or a derivative thereof, and M344; and

- the concentration of the flavonoid and M344 in a composition with the mammalian cell results in an increase of the genomic viral vector titer and the transducing viral vector titer by at least 10%, preferably at least 25%, compared to a viral vector production method without contacting the mammalian cell with the flavonoid and the M344.

According to a particular embodiment, the method according to the present disclosure is characterized by the following features:

- the mammalian cell is a human-derived cell line, preferably HEK293 or a derivative thereof;
- the mammalian cell is configured to produce a viral vector, preferably wherein the mammalian cell is modified by transiently transfecting the mammalian cell with one or more plasmids for viral vector production allowing for transient viral vector production;

- step (b) comprises contacting the mammalian cell with the flavonoid, preferably the flavonoid inhibits at least Cdk9, resulting in a concentration of at least 40 nM in a composition with the mammalian cell; and
- step (b) further comprises contacting the mammalian cell with the M344, preferably resulting in a concentration of at least 5 $\mu$M in a composition with the mammalian cell.

According to a particular embodiment, the method according to the present disclosure is characterized by the following features:

- step (a) comprises providing a mammalian cell configured to produce a viral vector, wherein the mammalian cell has a number of at least 1 million cells per mL;
- step (b) comprises contacting the mammalian cell with the flavonoid, preferably the flavonoid inhibits at least Cdk9, resulting in a concentration of at least 40 nM in a composition with the mammalian cell; and
- step (b) further comprises contacting the mammalian cell with the M344, preferably resulting in a concentration of at least 5 $\mu$M in a composition with the mammalian cell.

## Method according to a further aspect of the invention

According to a further aspect of the invention a viral vector production method is provided, comprising following steps:

(a) providing a mammalian cell configured to produce a viral vector;
(b) contacting the mammalian cell with a Cyclin-dependent Kinase (CdK) inhibitor;
(c) culturing the mammalian cell in presence of the CdK inhibitor to produce a viral vector; and
(d) optionally, harvesting the viral vector.

The method advantageously allows for improved viral vector production. By contacting the mammalian cell configured to produce a viral vector with the CdK inhibitor the mammalian cells are impacted such that a higher yield of viral vector and higher quality of viral vector can be produced. As demonstrated in the examples below, the inventors found that both the genomic but also the transducing viral vector titer can be markedly increased by contacting the mammalian cell with a flavonoid, e.g. flavopiridol, which are well-known CdK-inhibitors. As a result, the yield of viral vector that can be applicable for gene therapy is higher and of higher quality.

The individual steps and preferred embodiments of the method according to the further aspect correspond to the individual steps and embodiments of the method according to the first aspect, except for contacting step (b) and culturing step (c). For all other embodiments it is referred to the above disclosure which shall equally be applicable for the method according to the further aspect. This particularly but not exclusively includes the viral vector, the mammalian cell, providing step (a), further optional steps (d), (e), and (f), as well as the improvement in genomic and/or transducing titer.

Also, some aspects of steps (b) and (c) of the first aspect shall be applicable for the method according to the further aspect such as mode of culture, way of contacting, etc. The core differences between the method according to the first aspect and the method according to the further aspect relies in the CdK inhibitor in steps (b) and (c) compared to the flavonoid and/or M344. The individual steps and preferred embodiments of this particular difference will now be described in detail.

## CdK inhibitor

The method according to the further aspect comprises step (b) contacting the mammalian cell with a Cyclin-dependent Kinase (CdK) inhibitor and step (c) culturing the mammalian cell in presence of the CdK inhibitor to produce a viral vector. CdKs belong to the family of protein kinases and regulate the cell cycle. They are also involved in regulating transcription, mRNA processing, and the differentiation of nerve cells and are known in all known eukaryotes, and their regulatory function in the cell cycle has been evolutionarily conserved. A CdK inhibitor interacts with a cyclin-CdK complex to block kinase activity, usually during G1 or in response to signals from the environment or from damaged DNA. The present inventors found that by contacting the mammalian cell in step (b) with a CdK inhibitor and culturing the mammalian cell in presence of the CdK inhibitor improves the viral vector production. Specifically, the genomic and transducing viral vector can be improved by the CdK inhibitor flavopiridol as demonstrated in the Examples below (see Figs. 3, 4, 5, 11, 12A, 12B, 13).

Without being bound to theory, by inhibiting one or more CdKs of the mammalian cell the cell cycle progression is affected which positively affects also viral vector production. The CdK inhibitor could indirectly support viral DNA replication and lead to a higher transcription rate and higher packaging efficiency. According to a preferred embodiment, the CdK inhibitor inhibits one or more of CdK1, CdK2, Cdk3, CdK4, CdK5, CdK6, CdK7, CdK8, CdK9, CdK10, or CdK11.

It is further believed that from all CdKs, CdK9 may have a particular impact, such that its inhibition could further improve viral vector production. Accordingly in a preferred embodiment, the CdK inhibitor inhibits at least Cdk9.

According to a preferred embodiment, the CdK inhibitor is selected from one or more of the group comprising or consisting of flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

Contacting the mammalian cell in step (b) preferably results in a concentration of the CdK inhibitor in a composition with the mammalian cell that increases the genomic and/or transducing viral vector titer. In one embodiment, the concentration of the CdK inhibitor in a composition with the mammalian cell results in an increase of the genomic viral vector titer by at least 10%, preferably at least 25%, compared to a viral vector production method without contacting the mammalian cell with the CdK inhibitor. In one embodiment, the concentration of the CdK inhibitor in a composition with the mammalian cell results in an increase of the transducing viral vector titer by at least 10%, preferably at least 25%, compared to a viral vector production method without contacting the mammalian cell with the CdK inhibitor. In one embodiment, the concentration of the CdK inhibitor in a composition with the mammalian cell results in an increase of the genomic viral vector titer and the transducing viral vector titer by at least 10%, preferably at least 25%, compared to a viral vector production method without contacting the mammalian cell with the CdK inhibitor. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

According to a preferred embodiment, step (b) comprises contacting the mammalian cell with the CdK inhibitor resulting in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell. According to a further preferred embodiment, step (b) comprises contacting the mammalian cell with the CdK inhibitor resulting in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell. As demonstrated in the Examples below, such concentrations and concentration ranges lead to advantageously improved viral vector production. Specifically, such concentrations increase the genomic titer and/or increase the transducing titer in the disclosed viral vector production method (see Figs. 3, 4, 5, 11, 12A, 12B, and 13). The composition may comprise the mammalian cell, the CdK inhibitor and a cell culture medium. The composition may further comprise the solvent in which the CdK inhibitor is dissolved, e.g. an organic solvent, such as DMSO. The composition may comprise further compounds suitable for culturing the mammalian cell and/or producing the viral vector. In one embodiment, contacting step (b) may further comprise adding M344, optionally in a concentration which is disclosed above for the method according to the first aspect.

According to a particular embodiment, the method according to the further aspect comprises following features:

- the mammalian cell is a human-derived cell line, preferably HEK293 or a derivative thereof;
- the CdK inhibitor inhibits at least Cdk9;
- the concentration of the CdK inhibitor in a composition with the mammalian cell results in an increase of the genomic viral vector titer and the transducing viral vector titer by at least 10%, preferably at least 25%, compared to a viral vector production method without contacting the mammalian cell with the CdK inhibitor, wherein preferably contacting the CdK inhibitor results in a concentration of at least 40 nM in a composition with the mammalian cell; and
- the method comprises step (d).

**Method according to a further aspect of the invention**

According to a further aspect of the invention, a viral vector production method is provided, comprising following steps:

(a) providing a mammalian cell configured to produce a viral vector;
(b) contacting the mammalian cell with M344 resulting in a concentration of at least 5 $\mu$M M344 in a composition with the mammalian cell;
(c) culturing the mammalian cell in presence of the M344 to produce a viral vector; and
(d) optionally, harvesting the viral vector.

The method advantageously allows for improved viral vector production. By contacting the mammalian cell configured to produce a viral vector with the M344, resulting in a concentration of at least 5 $\mu$M M344 in a composition with the mammalian cell, the mammalian cells are impacted such that a higher yield of viral vector and higher quality of viral vector can be produced. As demonstrated in the examples below, the inventors found that both the genomic but also the transducing viral vector titer can be markedly increased by contacting the mammalian cell with M344. As a result, the yield of viral vector that can be applicable for gene therapy is higher and of higher quality.

The individual steps and preferred embodiments of the method according to the further aspect correspond to the individual steps and embodiments of the method according to the first aspect. Therefore, it is referred to the above disclosure which shall equally be applicable for the method according to the further aspect. This particularly but not exclusively includes the viral vector, the mammalian cell, providing step (a), contacting step (b), and culturing step (c), further optional steps (d), (e), and (f), as well as the improvement in genomic and/or transducing titer. Further features will now be described in detail.

M344, which is also referred to as 4-dimethylamino-N-(6-hydroxycarbamoylhexyl)-benzamide is an inhibitor of histone deacetylase (HDAC). HDACs remove acetyl moieties from lysine residues located on histones and cause changes in gene expression. This has been shown to reduce cancer growth (see Knoche et al., 2022, PLoS ONE 17(9): e0273518). HDAC inhibitors such as sodium butyrate and sodium valproate have been shown in the past to influence rAAV production, at high concentrations in the millimolar range (see WO 2020/033842 A1).

The inventors surprisingly found that the HDAC inhibitor M344 is very potent in improving the viral vector production, as is demonstrated in the examples below, especially for LV and AAV. It was shown that M344 improves the genomic and transducing viral vector titer. As demonstrated in the Examples below, such concentrations of at least 5 $\mu$M M344 in a composition with the mammalian cell lead to advantageously improved viral vector production. Specifically, such concentrations increase the genomic titer and/or increase the transducing titer in the disclosed viral vector production method (see Figs. 6, 7, 8, 9, 10, 11, 12A, 12B, and 13). Hence, according to a preferred embodiment, step (b) comprises contacting the mammalian cell with the M344 resulting in a concentration of at least 5 $\mu$M, at least 6 $\mu$M, at least 7 $\mu$M, at least 8 $\mu$M, at least 9 $\mu$M, at least 10 $\mu$M, preferably at least 5 $\mu$M in a composition with the mammalian cell. According to a further preferred embodiment, step (b) comprises contacting the mammalian cell with the M344 resulting in a concentration of 5 $\mu$M to 400 $\mu$M, 6 $\mu$M to 350 $\mu$M, 7 $\mu$M to 300 $\mu$M, 8 $\mu$M to 250 $\mu$M, 9 $\mu$M to 200 $\mu$M, 10 $\mu$M to 100 $\mu$M, preferably 5 $\mu$M to 100 $\mu$M in a composition with the mammalian cell.

In one embodiment, the concentration of the M344 in a composition with the mammalian cell results in an increase of the genomic viral vector titer by at least 10%, preferably at least 25%, compared to a viral vector production method without contacting the mammalian cell with the M344. In one embodiment, the concentration of the M344 in a composition with the mammalian cell results in an increase of the transducing viral vector titer by at least 10%, preferably at least 25%, compared to a viral vector production method without contacting the mammalian cell with the M344. In one embodiment, the concentration of the M344 in a composition with the mammalian cell results in an increase of the genomic viral vector titer and the transducing viral vector titer by at least 10%, preferably at least 25%, compared to a viral vector production method without contacting the mammalian cell with the M344. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

**System according to the second aspect of the invention**

According to a second aspect, a viral vector production system is provided, comprising:

- a mammalian cell configured to transiently or stably produce a viral vector, preferably the mammalian cell is a HEK293 cell or a derivative of a HEK293 cell; and
- a flavonoid and/or M344; and
- a cell culture medium for culturing the mammalian cell.

The viral vector production system according to the present invention advantageously allows for improved viral vector production. By comprising the mammalian cell configured to produce a viral vector and the flavonoid and/or M344 and a cell culture medium for culturing the mammalian cell, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the system according to the second aspect correspond to the embodiments of the method according to the first aspect. Therefore, it is referred to the above disclosure which shall equally be applicable for the system according to the second aspect. This particularly but not exclusively includes the viral vector, the mammalian cell, the flavonoid and/or M344, and the cell culture medium for culturing the mammalian cell, as well as the improvement in genomic and/or transducing titer. Further features will now be described in detail.

According to a preferred embodiment, the flavonoid is a flavone, preferably flavopiridol or a derivative thereof.

According to a preferred embodiment, the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof. According to a preferred embodiment, the flavonoid inhibits at least CdK9.

According to a preferred embodiment, the flavonoid is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

According to a preferred embodiment, the M344 is present in a concentration of at least 0.5 μM, at least 1 μM, at least 1.5 μM, at least 2 μM, at least 2.5 μM, at least 3 μM, at least 4 μM, at least 5 μM, at least 6 μM, at least 7 μM, at least 8 μM, at least 9 μM, at least 10 μM, preferably at least 5 μM.

According to a preferred embodiment, the system comprises a flavonoid, preferably flavopiridol, and M344.

According to a preferred embodiment, the genomic viral vector titer and/or transducing viral vector titer is increased compared to a viral vector production system without the flavonoid and/or the M344. According to a preferred embodiment, the genomic viral vector titer is increased by at least 10%, preferably at least 25%, compared to a viral vector production system without the flavonoid and/or the M344. According to a preferred embodiment, the transducing viral vector titer is increased by at least 10%, preferably at least 25%, compared to a viral vector production system without the flavonoid and/or the M344. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

According to a preferred embodiment, the viral vector is a lentivirus or an adeno-associated virus (AAV).

According to a preferred embodiment, the mammalian cell is selected from a HEK293 cell or a derivative of a HEK293 cell. According to a preferred embodiment, the HEK293 cell or the derivative of a HEK293 cell has been adapted for suspension culture.

The system may also be provided in form of a kit.

## System according to a further aspect of the invention

According to a further aspect, a viral vector production system is provided, comprising:

- a mammalian cell configured to transiently or stably produce a viral vector, preferably the mammalian cell is a HEK293 cell or a derivative of a HEK293 cell; and
- a cyclin-dependent kinase (CdK) inhibitor; and
- a cell culture medium for culturing the mammalian cell.

The viral vector production system according to the present invention advantageously allows for improved viral vector production. By comprising the mammalian cell configured to produce a viral vector and the cyclin-dependent kinase (CdK) inhibitor and a cell culture medium for culturing the mammalian cell, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the system according to the further aspect correspond to the embodiments of the system according to the second aspect (referring back to the method according to the first aspect), as well as the method according to the further aspect relating to the CdK inhibitor disclosed above. Therefore, it is referred to the above disclosure which shall equally be applicable for the system according to the second aspect. This particularly but not exclusively includes the viral vector, the mammalian cell, the cyclin-dependent kinase (CdK) inhibitor, and the cell culture medium for culturing the mammalian cell, as well as the improvement in genomic and/or transducing titer. Further features will now be described in detail.

According to a preferred embodiment, the CdK inhibitor inhibits one or more of CdK1, CdK2, Cdk3, CdK4, CdK5, CdK6, CdK7, CdK8, CdK9, CdK10, or CdK11 or a combination thereof. According to a preferred embodiment, the CdK inhibitor inhibits at least CdK9. According to a preferred embodiment, the CdK inhibitor is selected from one or more of the group comprising or consisting of flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

According to a preferred embodiment, the CdK inhibitor is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell. According to a preferred embodiment, the CdK inhibitor is present in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell.

The system may also be provided in form of a kit.

**System according to a further aspect of the invention**

According to a further aspect, a viral vector production system is provided, comprising:

- a mammalian cell configured to transiently or stably produce a viral vector, preferably the mammalian cell is a HEK293 cell or a derivative of a HEK293 cell; and
- M344 present in a concentration of at least 5 $\mu$M; and
- a cell culture medium for culturing the mammalian cell.

The viral vector production system according to the present invention advantageously allows for improved viral vector production. By comprising the mammalian cell configured to produce a viral vector and the M344 present in a concentration of at least 5 $\mu$M and a cell culture medium for culturing the mammalian cell, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the system according to the further aspect correspond to the embodiments of the system according to the second aspect (referring back to the method according to the first aspect), as well as the method according to the further aspect relating to the M344 present in a concentration of at least 5 $\mu$M disclosed above. Therefore, it is referred to the above disclosure which shall equally be applicable for the system according to the second aspect. This particularly but not exclusively includes the viral vector, the mammalian cell, the M344, and the cell culture medium for culturing the mammalian cell, as well as the improvement in genomic and/or transducing titer.

The system may also be provided in form of a kit.

**Composition according to the third aspect of the invention**

According to a third aspect, a viral vector production composition is provided, comprising:

- a mammalian cell configured to transiently or stably produce a viral vector, preferably the mammalian cell is a HEK293 cell or a derivative of a HEK293 cell; and
- a flavonoid and/or M344;

wherein the flavonoid and/or M344 increases production of the genomic viral vector titer and/or transducing viral vector titer compared to a composition without the flavonoid and/or M344.

The viral vector production composition according to the present invention advantageously allows for improved viral vector production. By comprising a mammalian cell configured to transiently or stably produce a viral vector, preferably the mammalian cell is a HEK293 cell or a derivative of a HEK293 cell and a flavonoid and/or M344, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the composition according to the third aspect correspond to the embodiments of the method according to the first aspect. Therefore, it is referred to the above disclosure which shall equally be applicable for the composition according to the third aspect. This particularly but not exclusively includes the viral vector, the mammalian cell, the flavonoid and/or M344, as well as the improvement in genomic and/or transducing titer. Further features will now be described in detail.

According to a preferred embodiment, the flavonoid is a flavone, preferably flavopiridol or a derivative thereof. According to a preferred embodiment, the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof. According to a preferred embodiment, wherein the flavonoid inhibits at least CdK9.

According to a preferred embodiment, the flavonoid is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

According to a preferred embodiment, the M344 is present in a concentration of at least 0.5 $\mu$M, at least 1 $\mu$M, at least 1.5 $\mu$M, at least 2 $\mu$M, at least 2.5 $\mu$M, at least 3 $\mu$M, at least 4 $\mu$M, at least 5 $\mu$M, at least 6 $\mu$M, at least 7 $\mu$M, at least 8 $\mu$M, at least 9 $\mu$M, at least 10 $\mu$M, preferably at least 5 $\mu$M.

According to a preferred embodiment, the system comprises a flavonoid, preferably flavopiridol, and M344.

According to a preferred embodiment, the genomic viral vector titer increases by at least 10%, preferably at least 25%. According to a preferred embodiment, the transducing viral vector titer increases by at least 10%, preferably at least 25%. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

According to a preferred embodiment, the composition further comprises a cell culture medium for culturing the mammalian cells.

## Composition according to a further aspect of the invention

According to a further aspect, a viral vector production composition is provided, comprising:

-  a mammalian cell configured to transiently or stably produce a viral vector, preferably the mammalian cell is a HEK293 cell or a derivative of a HEK293 cell; and
-  a cyclin-dependent kinase (CdK) inhibitor;

wherein the flavonoid and/or M344 increases production of the genomic viral vector titer and/or transducing viral vector titer compared to a composition without the flavonoid and/or M344.

The viral vector production composition according to the present invention advantageously allows for improved viral vector production. By comprising a mammalian cell configured to transiently or stably produce a viral vector, preferably the mammalian cell is a HEK293 cell or a derivative of a HEK293 cell and a cyclin-dependent kinase (CdK) inhibitor, such as flavopiridol, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the composition according to the further aspect correspond to the embodiments of the composition according to the third aspect (referring back to the method according to the first aspect), as well as the method according to the further aspect relating to the CdK inhibitor disclosed above. Therefore, it is referred to the above disclosure which shall equally be applicable for the composition according to the further aspect. This particularly but not exclusively includes the viral vector, the mammalian cell, the cyclin-dependent kinase (CdK) inhibitor, and the cell culture medium for culturing the mammalian cell, as well as the improvement in genomic and/or transducing titer. Further features will now be described in detail.

According to a preferred embodiment, the CdK inhibitor inhibits one or more of CdK1, CdK2, Cdk3, CdK4, CdK5, CdK6, CdK7, CdK8, CdK9, CdK10, and CdK11. According to a preferred embodiment, the CdK inhibitor inhibits at least CdK9. According to a preferred embodiment, the CdK inhibitor is selected from one or more of the group comprising flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, P-276-00, and any derivative of the aforementioned.

According to a preferred embodiment, the CdK inhibitor is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell. According to a preferred embodiment, the CdK inhibitor is present in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell.

## Composition according to a further aspect of the invention

According to a further aspect, a viral vector production composition is provided, comprising:

-  a mammalian cell configured to transiently or stably produce a viral vector, preferably the mammalian cell is a HEK293 cell or a derivative of a HEK293 cell; and
-  M344 present in a concentration of at least 5 $\mu$M;

wherein the flavonoid and/or M344 increases production of the genomic viral vector titer and/or transducing viral vector titer compared to a composition without the flavonoid and/or M344.

The viral vector production composition according to the present invention advantageously allows for improved viral vector production. By comprising a mammalian cell configured to transiently or stably produce a viral vector, preferably the mammalian cell is a HEK293 cell or a derivative of a HEK293 cell and M344 present in a concentration of at least 5 μM, the mammalian cells are impacted such that a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the composition according to the further aspect correspond to the embodiments of the composition according to the third aspect (referring back to the method according to the first aspect), as well as the method according to the further aspect relating to the M344 present in a concentration of at least 5 μM disclosed above. Therefore, it is referred to the above disclosure which shall equally be applicable for the composition according to the further aspect. This particularly but not exclusively includes the viral vector, the mammalian cell, the M344, and the cell culture medium for culturing the mammalian cell, as well as the improvement in genomic and/or transducing titer.

## Use according to the fourth aspect of the invention

According to a fourth aspect, flavonoid for use for increased transient or stable viral vector production is provided.

Flavonoid for use for increased transient or stable viral vector production is provided according to the present invention which advantageously allows for improved viral vector production. The flavonoid results in a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the use according to the fourth aspect correspond to the embodiments of the method according to the first aspect. Therefore, it is referred to the above disclosure which shall equally be applicable for the use according to the fourth aspect. This particularly but not exclusively includes the viral vector, the mammalian cell, and the flavonoid, as well as the improvement in genomic and/or transducing titer. Further features will now be described in detail.

According to a preferred embodiment, the flavonoid is a flavone, preferably flavopiridol or a derivative thereof. According to a preferred embodiment, the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof. According to a preferred embodiment, the flavonoid inhibits at least CdK9.

According to a preferred embodiment, the flavonoid is used in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

According to a preferred embodiment, the use increases the genomic viral vector titer and/or transducing viral vector titer. According to a preferred embodiment, the use increases the genomic viral vector titer is by at least 10%, preferably at least 25%. According to a preferred embodiment, the use increases the transducing viral vector titer by at least 10%, preferably at least 25%. Methods of measuring the genomic and transducing viral vector titer are available in the art and known by the person skilled in the art. In a particular embodiment, the genomic viral vector titer is measured by droplet digital PCR. The transducing titer which may also be referred to as the infectious titer or infection titer may be measured by a transduction assay.

According to a preferred embodiment, the transient or stable viral vector is a lentivirus or an adeno-associated virus (AAV).

## Use according to a further aspect of the invention

According to a further aspect, cyclin-dependent kinase (CdK) inhibitor for use for increased transient or stable viral vector production is provided.

CdK inhibitor for use for increased transient or stable viral vector production is provided according to the present invention which advantageously allows for improved viral vector production. The CdK inhibitor results in a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the use according to the further aspect correspond to the embodiments of the method according to the first aspect, as well as the method according to the further aspect relating to the CdK inhibitor disclosed above. Therefore, it is referred to the above disclosure which shall equally be applicable for the use according to the further aspect. This particularly but not exclusively includes the viral vector, the cyclin-dependent kinase (CdK) inhibitor, as well as the improvement in genomic and/or transducing titer. Further features will now be described in detail.

According to a preferred embodiment, the CdK inhibitor inhibits one or more of CdK1, CdK2, Cdk3, CdK4, CdK5, CdK6, CdK7, CdK8, CdK9, CdK10, and CdK11. According to a preferred embodiment, the CdK inhibitor inhibits at least CdK9. According to a preferred embodiment, the CdK inhibitor is selected from one or more of the group comprising flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

According to a preferred embodiment, the CdK inhibitor is used in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM. According to a preferred embodiment, the CdK inhibitor is used in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM.

## Use according to a further aspect of the invention

According to a further aspect, M344 for use for increased transient or stable viral vector production, wherein M344 is used in a concentration of at least 5 μM is provided.

M344 for use for increased transient or stable viral vector production is provided according to the present invention which advantageously allows for improved viral vector production. M344 results in a higher yield of viral vector and higher quality of viral vector can be produced. As demonstrated in the examples below, the inventors found that both the genomic but also the transducing viral vector titer can be markedly increased by the production system. As a result, the yield of viral vector that can be applicable for gene therapy is higher and of higher quality.

The individual parts and preferred embodiments of the use according to the further aspect correspond to the embodiments of the method according to the first aspect, as well as the method according to the further aspect relating to the M344 disclosed above. Therefore, it is referred to the above disclosure which shall equally be applicable for the use according to the further aspect. This particularly but not exclusively includes the viral vector, M344, as well as the improvement in genomic and/or transducing titer.

## Items according to the present disclosure

The following items provide further advantageous embodiments of the present disclosure:

1. A viral vector production method, comprising following steps:

(a) providing a mammalian cell configured to produce a viral vector;
(b) contacting the mammalian cell with a flavonoid and/or M344;
(c) culturing the mammalian cell in presence of the flavonoid and/or M344 to produce a viral vector; and
(d) optionally, harvesting the viral vector.

2. The method according to item 1, wherein the flavonoid is selected from the group consisting of isoflavones, neoflavonoids, flavones, flavonols, flavanones, flavanonols, flavanols, catechins, anthocyanins and chalcones, preferably the flavonoid is a flavone.

3. The method according to item 1 or 2, wherein the flavonoid is chemically synthesized, preferably a chemically synthesized flavone.

4. The method according to one or more of items 1 to 3, wherein the flavonoid is selected from one or more of the group consisting of flavopiridol, catechin, genistein, quercetin, wogonin, kaempferol, baicalein, baicalin, fisetin, apigenin, luteolin, chrysin, rohitukine, P-276-00, and any derivative of the aforementioned.

5. The method according to one or more of items 1 to 4, wherein the flavonoid is flavopiridol or a derivative thereof.

6. The method according to one or more of items 1 to 5, wherein the flavonoid inhibits one or more cyclin-dependent kinases (CdK), preferably selected from Cdk1, Cdk2, Cdk3, Cdk4, Cdk5, Cdk6, Cdk7, Cdk8, Cdk9, Cdk10, Cdk11, or a combination thereof.

7. The method according to one or more of items 1 to 6, wherein the flavonoid inhibits at least Cd k9.

8. The method according to one or more of items 1 to 7, wherein step (b) comprises contacting the mammalian cell with the flavonoid resulting in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell.

9. The method according to one or more of items 1 to 8, wherein step (b) comprises contacting the mammalian cell with the flavonoid resulting in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell.

10. The method according to one or more of items 1 to 9, wherein step (b) comprises contacting the mammalian cell with the flavonoid resulting in a concentration of at least 40 nM in the composition with the mammalian cell, and wherein the flavonoid inhibits at least CdK9.

11. The method according to one or more of items 1 to 10, wherein step (b) comprises contacting the mammalian cell with the flavonoid resulting in a concentration of at least 40 nM in the composition with the mammalian cell, and wherein the flavonoid is flavopiridol or a derivative thereof.

12. The method according to one or more of items 1 to 11, wherein step (b) comprises contacting the mammalian cell with the M344 resulting in a concentration of at least 0.5 $\mu$M, at least 1 $\mu$M, at least 1.5 $\mu$M, at least 2 $\mu$M, at least 2.5 $\mu$M, at least 3 $\mu$M, at least 4 $\mu$M, at least 5 $\mu$M, at least 6 $\mu$M, at least 7 $\mu$M, at least 8 $\mu$M, at least 9 $\mu$M, at least 10 $\mu$M, preferably at least 5 $\mu$M in a composition with the mammalian cell.

13. The method according to one or more of items 1 to 12, wherein step (b) comprises contacting the mammalian cell with the flavonoid, preferably flavopiridol, and the M344.

14. A viral vector production method, comprising following steps:

(a) providing a mammalian cell configured to produce a viral vector;
(b) contacting the mammalian cell with a Cyclin-dependent Kinase (CdK) inhibitor;
(c) culturing the mammalian cell in presence of the CdK inhibitor to produce a viral vector; and
(d) optionally, harvesting the viral vector.

15. The method according to item 14, wherein the CdK inhibitor inhibits one or more of CdK1, CdK2, Cdk3, CdK4, CdK5, CdK6, CdK7, CdK8, CdK9, CdK10, or CdK11.

16. The method according to item 14 or 15, wherein the CdK inhibitor inhibits at least CdK9.

17. The method according to one or more of items 14 to 16, wherein the CdK inhibitor is selected from one or more of the group comprising or consisting of flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

18. The method according to one or more of items 14 to 17, wherein step (b) comprises contacting the mammalian cell with the CdK inhibitor resulting in a concentration of the CdK inhibitor of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell.

19. The method according to one or more of items 14 to 18, wherein step (b) comprises contacting the mammalian cell with the CdK inhibitor resulting in a concentration of the CdK inhibitor of 1 nM to 1000 nM, 2 nM to 900 nM, 3 nM to 800 nM, 4 nM to 700 nM, or 5 nM to 600 nM, preferably 10 nM to 500 nM, more preferably 40 nM to 500 nM in a composition with the mammalian cell.

20. A viral vector production method, comprising following steps:

(a) providing a mammalian cell configured to produce a viral vector;
(b) contacting the mammalian cell with M344 resulting in a concentration of at least 5 $\mu$M M344 in a composition with the mammalian cell;
(c) culturing the mammalian cell in presence of the M344 to produce a viral vector; and
(d) optionally, harvesting the viral vector.

21. The method according to one or more of items 1 to 20, wherein the genomic viral vector titer and/or transducing viral vector titer is increased compared to a viral vector production method without contacting the mammalian cell with the flavonoid and/or the M344.

22. The method according to item 21, wherein the genomic viral vector titer is increased by at least 10%, preferably at least 25%, compared to a viral vector production method without contacting the mammalian cell with the flavonoid and/or the M344.

23. The method according to item 21 or 22, wherein the transducing viral vector titer is increased by at least 10%, preferably at least 25%, compared to a viral vector production method without contacting the mammalian cell with the flavonoid and/or M344.

24. The method according to one or more of items 1 to 23, wherein the viral vector is a lentivirus or an adeno-associated virus (AAV).

25. The method according to one or more of items 1 to 24, wherein the mammalian cell is modified to be configured to produce a viral vector.

26. The method according to item 25, wherein the mammalian cell is modified by:

(I) transiently transfecting the mammalian cell with one or more plasmids for viral vector production allowing for transient viral vector production; or
(II) genome editing the mammalian cell with one or more nucleic acid molecules for viral vector production allowing for stable viral vector production.

27. The method according to item 26, wherein transiently transfecting the mammalian cell comprises transfecting with at least 0.1 μg plasmid DNA per 1 million cells, preferably at least 0.5 μg or at least 1 μg plasmid DNA per 1 million cells.

28. The method according to item 26 or 27, wherein transiently transfecting comprises:

(x) combining the mammalian cell with the one or more plasmids for viral vector production and a transfection reagent; and
(y) incubating the mammalian cell, the one or more plasmids for viral vector production and the transfection reagent to generate a transiently transfected cell.

29. The method according to item 28, wherein step (x) comprises one of the following:

(x.1)

i. combining a cell culture medium with the one or more plasmids for viral vector production and the transfection reagent to form a transfection composition;
ii. optionally, mixing the transfection composition of (i);
iii. optionally, incubating the transfection composition of (i) or (ii);
iv. combining the transfection composition of (i), (ii) or (iii) and the mammalian cell; and
v. optionally, the transfection composition of (i), (ii), or (iii) or the composition of (iv) further comprises the flavonoid and/or M344; or

(x.2)

i. adding the one or more plasmids for viral vector production and the transfection reagent to the mammalian cell present in a cell culture medium, optionally further comprising the flavonoid and/or M344.

30. The method according to item 28 or 29, wherein step (y) comprises incubating for at least 1 hour, at least 2 hours, at least 3 hours, preferably at least 4 hours.

31. The method according to one or more of items 28 to 30, wherein step (y) comprises incubating for 1 to 48 hours, 2 to 36 hours, 3 to 24 hours, preferably 4 to 12 hours, more preferably 4 to 8 hours or 4 to 6 hours.

32. The method according to one or more of items 28 to 31, wherein the transfection reagent is polymer-based or lipid-based, preferably being cationic, such as polyethylene imine or a derivative thereof.

33. The method according to item 26, wherein genome editing the mammalian cell comprises stably integrating the one or more nucleic acid molecules into the chromosome of the mammalian cell to be capable of stably producing a viral vector.

34. The method according to item 33, wherein the one or more nucleic acid molecules comprise an induction system, such as one or more inducible promotor.

35. The method according to one or more of items 26 to 35, wherein the one or more plasmids or the one or more nucleic acid molecules for viral vector production encode at least part of a lentivirus or at least part of an adeno-associated virus (AAV).

36. The method according to item 35, wherein the one or more plasmids or the one or more nucleic acid molecules for viral vector production encode one or more of the group comprising long terminal repeats (LTRs), polypurine tract (PPT), Rev response element (RRE), psi packaging signal, HIV-1 gag-pol, rev proteins, and enveloped glycoprotein, preferably all of the aforementioned, for producing a lentivirus.

37. The method according to item 35, wherein the one or more plasmids or the one or more nucleic acid molecules for viral vector production encode one or more of the group comprising Rep78, Rep68, Rep52, Rep40, VP1, VP2, VP3, ITR, AAP, MAAP, X Gene, VA RNA, E4orf6, and E2A, preferably all of the aforementioned, for producing an AAV.

38. The method according to one or more of items 1 to 37, wherein the flavonoid and/or M344 is/are dissolved prior to contacting step (b).

39. The method according to item 38, wherein the flavonoid and/or M344 is/are dissolved in an organic solvent, preferably dimethyl sulfoxide (DMSO), ethanol, dimethyl formamide (DMF), or chloroform, more preferably DMSO.

40. The method according to one or more of items 1 to 39, wherein step (c) comprises culturing the mammalian cell for at least 12 hours, at least 18 hours, at least 24 hours, at least 36 hours, preferably at least 48 hours, more preferably at least 72 hours.

41. The method according to one or more of items 1 to 40, wherein:

- step (a) further comprises culturing the mammalian cell to generate at least 1 million viable cells per mL, preferably at least 2 million viable cells per mL, more preferably at least 3 million cells per mL; and/or
- step (a) comprises providing a mammalian cell configured to produce a viral vector, wherein the mammalian cell has a number of at least 0.5 million cells per mL, at least 0.75 million cells per mL, or preferably at least 1 million cells per mL.

42. The method according to one or more of items 1 to 41, wherein the mammalian cell is selected from a HEK293 cell or a derivative of a HEK293 cell.

43. The method according to item 42, wherein the HEK293 cell or the derivative of a HEK293 cell has been adapted for suspension culture.

44. The method according to one or more of items 1 to 43, wherein step (a) further comprises culturing the mammalian cell in suspension culture.

45. The method according to one or more of items 1 to 44, wherein step (c) comprises culturing the mammalian cell in suspension culture.

46. The method according to one or more of items 1 to 45, wherein the viral vector is harvested in step (d).

47. The method according to item 46, wherein harvesting comprises lysing the transiently transfected cells.

48. The method according to one or more of items 1 to 47, wherein the method further comprises step (e) purifying the harvested viral vector.

49. The method according to item 48, wherein the method further comprises step (f) formulating the viral vector for gene therapy.

50. A viral vector production system, comprising:

- a mammalian cell configured to transiently or stably produce a viral vector, preferably the mammalian cell is a HEK293 cell or a derivative of a HEK293 cell; and
- a flavonoid and/or M344; and
- a cell culture medium for culturing the mammalian cell.

51. The system according to item 50, wherein the flavonoid is a flavone, preferably flavopiridol or a derivative thereof.

52. The system according to item 50 or 51, wherein the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof.

53. The system according to one or more of items 50 to 52, wherein the flavonoid inhibits at least Cd K9.

54. The system according to one or more of items 50 to 53, wherein the flavonoid is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

55. The system according to one or more of items 50 to 54, wherein the M344 is present in a concentration of at least 0.5 μM, at least 1 μM, at least 1.5 μM, at least 2 μM, at least 2.5 μM, at least 3 μM, at least 4 μM, at least 5 μM, at least 6 μM, at least 7 μM, at least 8 μM, at least 9 μM, at least 10 μM, preferably at least 5 μM.

56. The system according to one or more of items 50 to 55, wherein the system comprises a flavonoid, preferably flavopiridol, and M344.

57. A viral vector production system, comprising:

- a mammalian cell configured to transiently or stably produce a viral vector, preferably the mammalian cell is a HEK293 cell or a derivative of a HEK293 cell; and
- a cyclin-dependent kinase (CdK) inhibitor; and
- a cell culture medium for culturing the mammalian cell.

58. The system according to item 57, wherein the CdK inhibitor inhibits one or more of CdK1, CdK2, Cdk3, CdK4, CdK5, CdK6, CdK7, CdK8, CdK9, CdK10, or CdK11 or a combination thereof.

59. The system according to item 57 or 58, wherein the CdK inhibitor inhibits at least CdK9.

60. The system according to one or more of items 57 to 59, wherein the CdK inhibitor is selected from one or more of the group comprising or consisting of flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

61. The system according to one or more of items 57 to 60, wherein the CdK inhibitor is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell.

62. The system according to one or more of items 57 to 61, wherein the CdK inhibitor is present in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell.

63. A viral vector production system, comprising:

- a mammalian cell configured to transiently or stably produce a viral vector, preferably the mammalian cell is a HEK293 cell or a derivative of a HEK293 cell; and
- M344 present in a concentration of at least 5 $\mu$M; and
- a cell culture medium for culturing the mammalian cell.

64. The system according to one or more of items 50 to 63, wherein the genomic viral vector titer and/or transducing viral vector titer is increased compared to a viral vector production system without the flavonoid and/or the M344.

65. The system according to item 64, wherein the genomic viral vector titer is increased by at least 10%, preferably at least 25%, compared to a viral vector production system without the flavonoid and/or the M344.

66. The system according to item 64 or 65, wherein the transducing viral vector titer is increased by at least 10%, preferably at least 25%, compared to a viral vector production system without the flavonoid and/or the M344.

67. The system according to one or more of items 50 to 66, wherein the viral vector is a lentivirus or an adeno-associated virus (AAV).

68. The system according to one or more of items 50 to 67, wherein the mammalian cell is selected from a HEK293 cell or a derivative of a HEK293 cell.

69. The system according to item 68, wherein the HEK293 cell or the derivative of a HEK293 cell has been adapted for suspension culture.

70. A viral vector production composition, comprising:

- a mammalian cell configured to transiently or stably produce a viral vector, preferably the mammalian cell is a HEK293 cell or a derivative of a HEK293 cell; and
- a flavonoid and/or M344;

wherein the flavonoid and/or M344 increases production of the genomic viral vector titer and/or transducing viral vector titer compared to a composition without the flavonoid and/or M344.

71. The composition according to item 70, wherein the flavonoid is a flavone, preferably flavopiridol or a derivative thereof.

72. The composition according to item 70 or 71, wherein the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof.

73. The composition according to one or more of items 70 to 72, wherein the flavonoid inhibits at least CdK9.

74. The composition according to one or more of items 70 to 73, wherein the flavonoid is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

75. The composition according to one or more of items 70 to 74, wherein the M344 is present in a concentration of at least 0.5 pM, at least 1 pM, at least 1.5 pM, at least 2 pM, at least 2.5 pM, at least 3 pM, at least 4 pM, at least 5 pM, at least 6 pM, at least 7 pM, at least 8 $\mu$M, at least 9 pM, at least 10 $\mu$M, preferably at least 5 $\mu$M.

76. The composition according to one or more of items 74 to 75, wherein the system comprises a flavonoid, preferably flavopiridol, and M344.

77. A viral vector production composition, comprising:

- a mammalian cell configured to transiently or stably produce a viral vector, preferably the mammalian cell is a HEK293 cell or a derivative of a HEK293 cell; and
- a cyclin-dependent kinase (CdK) inhibitor;

wherein the CdK inhibitor increases production of the genomic viral vector titer and/or transducing viral vector titer compared to a composition without the CdK inhibitor.

78. The composition according to item 77, wherein the CdK inhibitor inhibits one or more of CdK1, CdK2, Cdk3, CdK4, CdK5, CdK6, CdK7, CdK8, CdK9, CdK10, and CdK11.

79. The composition according to item 77 or 78, wherein the CdK inhibitor inhibits at least CdK9.

80. The composition according to one or more of items 77 to 79, wherein the CdK inhibitor is selected from one or more of the group comprising flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, P-276-00, and any derivative of the aforementioned.

81. The composition according to one or more of items 77 to 80, wherein the CdK inhibitor is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell.

82. The composition according to one or more of items 77 to 81, wherein the CdK inhibitor is present in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM in a composition with the mammalian cell.

83. A viral vector production composition, comprising:

- a mammalian cell configured to transiently or stably produce a viral vector, preferably the mammalian cell is a HEK293 cell or a derivative of a HEK293 cell; and
- M344 present in a concentration of at least 5 pM; and

wherein the M344 increases production of the genomic viral vector titer and/or transducing viral vector titer compared to a composition without the flavonoid and/or M344.

84. The composition according to one or more of items 70 to 83, wherein the genomic viral vector titer increases by at least 10%, preferably at least 25%.

85. The composition according to one or more of items 70 to 84, wherein the transducing viral vector titer increases by at least 10%, preferably at least 25%.

86. The composition according to one or more of items 74 to 85, wherein the composition further comprises a cell culture medium for culturing the mammalian cells.

87. Flavonoid for use for increased transient or stable viral vector production.

88. The flavonoid for use according to item 87, wherein the flavonoid is a flavone, preferably flavopiridol or a derivative thereof.

89. The flavonoid for use according to item 87 or 88, wherein the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof.

90. The flavonoid for use according to one or more of items 87 to 89, wherein the flavonoid inhibits at least CdK9.

91. The flavonoid for use according to one or more of items 87 to 90, wherein the flavonoid is used in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

92. Cyclin-dependent kinase (CdK) inhibitor for use for increased transient or stable viral vector production.

93. The CdK inhibitor for use according to item 92, wherein the CdK inhibitor inhibits one or more of CdK1, CdK2, Cdk3, CdK4, CdK5, CdK6, CdK7, CdK8, CdK9, CdK10, and CdK11.

94. The CdK inhibitor for use according to item 92 or 93, wherein the CdK inhibitor inhibits at least CdK9.

95. The CdK inhibitor for use according to one or more of items 92 to 94, wherein the CdK inhibitor is selected from one or more of the group comprising flavopiridol, NVP-2, JSH-150, BAY 1251152, SNS-032 (BMS-387032), Dinaciclib (SCH727965), AZD4573, MC180295, CDKI-73, KB-0742 Dihydrochloride, CDK-IN-2, AT7519, AT7519 HCl, LY2857785, CGP60474, Atuveciclib (BAY-1143572), Riviciclib hydrochloride (P276-00), Catechin, Genistein, Quercetin (NSC 9221), Wogonin, Kaempferol, baicalein, baicalin, fisetin, luteolin, chrysin, rohitukine, and any derivative of the aforementioned.

96. The CdK inhibitor for use according to one or more of items 92 to 95, wherein the CdK inhibitor is used in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

97. The CdK inhibitor for use according to one or more of items 92 to 96, wherein the CdK inhibitor is used in a concentration of 1 nM to 1000 nM, 2 nM to 900 nM, 3 nM to 800 nM, 4 nM to 700 nM, 5 nM to 600 nM, preferably 10 nM to 500 nM, more preferably 40 nM to 500 nM.

98. M344 for use for increased transient or stable viral vector production, wherein M344 is used in a concentration of at least 5 $\mu$M.

99. Flavonoid for use according to one or more of items 87 to 91 or CdK inhibitor for use according to one or more of items 92 to 97 or M344 for use according to item 98, wherein the use increases the genomic viral vector titer and/or transducing viral vector titer.

100. Flavonoid, CdK inhibitor or M344 for use according to item 99, wherein the use increases the genomic viral vector titer is by at least 10%, preferably at least 25%.

101. Flavonoid, CdK inhibitor or M344 for use according to item 99 or 100, wherein the use increases the transducing viral vector titer by at least 10%, preferably at least 25%.

102. Flavonoid for use according to one or more of items 87 to 91 or 99 to 101 or CdK inhibitor for use according to one or more of items 92 to 97 or 99 to 101 or M344 for use according to one or more of items 98 or 99 to 101, wherein the transient or stable viral vector is a lentivirus or an adeno-associated virus (AAV).

103. Flavonoid for use according to one or more of items 87 to 91 or 99 to 101 or CdK inhibitor for use according to one or more of items 92 to 97 or 99 to 101 or M344 for use according to one or more of items 98 or 99 to 101, wherein the flavonoid or CdK inhibitor or M344 is used in a method according to one or more of claims 1 to 49.

104. The viral vector production system according to one or more of items 50 to 69 or the viral vector production composition according to one or more of items 70 to 86, wherein the system or composition is used in a method according to one or more of claims 1 to 49.

Throughout the description, where methods, systems, compositions or uses are described as having, including, or comprising specific components or steps, it is contemplated that, additionally, there are methods, systems, compositions or uses of the present invention that consist essentially of, or consist of, the recited components or steps.

In the application, where an element or component is said to be included in and/or selected from a list of recited elements or components, it should be understood that the element or component can be any one of the recited elements or components, or the element or component can be selected from a group consisting of two or more of the recited elements or components.

Terms "a" and "an" and "the" and similar reference used in the context of describing the invention (especially in the context of the claims) are to be construed to cover both the singular and the plural, unless otherwise indicated herein or clearly contradicted by context.

The use of the term "include," "includes," "including," "have," "has," "having," "contain," "contains," or "containing," including grammatical equivalents thereof, should be understood generally as openended and non-limiting, for example, not excluding additional unrecited elements or steps, unless otherwise specifically stated or understood from the context.

Where the use of the term "about" or "approximately" is before a quantitative value, the present invention also includes the specific quantitative value itself, unless specifically stated otherwise. As used herein, the term "about" refers to a $\pm 10\%$ variation from the nominal value unless otherwise indicated or inferred.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

All citations are hereby incorporated by reference.

All individual embodiments and aspects as disclosed herein can be combined with each other within the framework and context of the present disclosure. It will be understood that the embodiments disclosed herein are only exemplary, and that any feature presented for a particular exemplary embodiment may be used with the present disclosure on its own or in combination with any feature presented for the same or another particular exemplary embodiment and/or in combination with any other feature not mentioned. It will further be understood that any feature presented for an example embodiment in a particular category may also be used in a corresponding manner in an example embodiment of any other category.

**EXAMPLES**

It should be understood that the following examples are for illustrative purpose only and are not to be construed as limiting this invention in any manner. The following examples demonstrate the advantageous improved viral vector production using the compounds ("enhancers") identified by the inventors.

**Materials and Methods**

Routine cultivation of HEK293 cells:

FreeStyle 293-F cells, Viral Production Cells 2.0 and LV MAX Production Cells (all Thermo Fisher Scientific) were thawed and cultivated in HEK ViP NB medium (Sartorius Xell GmbH). A suspension-adapted HEK293 clone (Sartorius Stedim Cellca GmbH) was thawed and cultivated in HEK TF medium (Sartorius Xell GmbH). Cell culture was sub cultured and seeded at VCD of 0.25 to 0.4 million cells per mL every three to four days in 30 mL of fresh culture medium in plain bottom shake flasks with a vented cap (Corning). Cells were cultivated at 37 °C, 5 % $CO_2$ at 130 rpm (FreeStyle 293-F, Thermo Fisher Scientific) or 185 rpm (Viral Production Cells 2.0, LV MAX Production Cells (both Thermo Fisher Scientific) and suspension-adapted HEK293 clone) with 50 mm orbit. The tested mammalian cells are shown in the below table:

Table 1: HEK293 cell lines

| Name | Supplier (Cat.No.) |
| --- | --- |
| FreeStyle 293-F | Thermo Fisher Scientific (R79007) |
| Viral Production Cells 2.0 | Thermo Fisher Scientific (A49784) |
| Suspension-adapted HEK293 clone | Sartorius Stedim Cellca GmbH (N/A) |
| HEK293 (adherent) | ATCC (CRL-1573) |
| LV MAX Production Cells | Thermo Fisher Scientific (A35684) |
| HEK293FT ViraPower (adherent) | Thermo Fisher Scientific (10769564) |

Transient transfection of mammalian cells (HEK293) to produce rAAV in 24-well plates:

One day prior to transfection, cells were seeded in a shake flask at a VCD of 1 million cells per mL in 30 mL of the respective culture medium. On the day of transfection, the culture should have reached a VCD of 2 million cells per mL. Cell culture was diluted to 0.4 million cells per mL in 35 mL minus the volume of transfection mixture to be added.

Per 1 million cells, 1 µg of plasmid DNA (pDNA) is used. The rAAV production plasmids from a two-plasmid system (pAAV-ssGFP and pDG, both PlasmidFactory) were used in a molar ratio as instructed by the supplier (PlasmidFactory, see Table 2). PEI-MAX (Polysciences) was used as a transfection reagent in a 4:1 ratio regarding pDNA amount. Pre-complexation was done in the respective culture medium, in 10 % of the final culture volume (3.5 mL) minus calculated pDNA and PEI-MAX volumes. First, the pDNA was added to the culture medium. Afterwards, PEI-MAX was added, and the mixture was mixed in an overhead shaker for 20 seconds at 15 rpm. After an incubation phase of 30 minutes at room temperature, the transfection mixture was added to the culture and the shake flask placed back in the incubator. After 30 min of incubation 500 µL of transfected cell culture was dispensed into wells of a 24-well plate for further screening experiments.

Table 2: Plasmids

| Name | Supplier (Cat.No.) |
| --- | --- |
| pAAV-ssGFP (1 mg per mL) | Plasmid Factory (PF1451) |
| pDG (1 mg per mL) | Plasmid Factory (PF0421) |

Transient transfection of mammalian cells (HEK293) to produce rAAV in 125 mL-shake flasks:

One day prior to transfection, cells were seeded at a VCD of 1.5 million cells per mL in 22 mL of HEK ViP NB or HEK TF, respectively. On the day of transfection, the culture should have reached a VCD of 3 to 3.5 million cells per mL. To achieve the final VCD for transfection, 7 mL of fresh culture medium was added to the shake flask.

Per 1 million cells, 1 µg of pDNA is used. The rAAV production plasmids from a two-plasmid system (pAAV-ssGFP and pDG, both PlasmidFactory) were used in a molar ratio of 1:1.3. PEI-MAX (Polysciences) was used as a transfection reagent in a 4:1 ratio regarding pDNA amount. Pre-complexation was done in the respective culture medium, in 10 % of the final culture volume (3.0 mL) minus calculated pDNA and PEI-MAX volumes. First, the pDNA was added to the culture medium. Afterwards, PEI-MAX was added, and the mixture was mixed in an overhead shaker for 20 seconds at 15 rpm. After an incubation phase of 30 minutes at room temperature, the transfection mixture was added to the culture and the shake flask placed back in the incubator.

Transient transfection of mammalian cells (HEK293) to produce LV particles in 125 mL-shake flasks

One day prior to transfection, cells were seeded at a VCD of 1.9 million cells per mL in 22 mL of HEK ViP NB. On the day of transfection, the culture should have reached a VCD of 3.5 to 4.0 million cells per mL. To achieve the final VCD for transfection, 8.5 mL of fresh culture medium was added to the shake flask.

Per 1 million cells, 1 $\mu$g of pDNA is used. The LV particles production plasmids from a four-plasmid system were used in ratio of VSV-G, GagPol, Rev, eGFP as instructed by the supplier (Aldevron, Oxgene). PEI-MAX was used as a transfection reagent in a 4:1 ratio regarding pDNA amount. Pre-complexation was done in the respective culture medium, in 5 % of the final culture volume (1.5 mL) minus calculated pDNA and PEI-MAX volumes. First, the pDNA was added to the culture medium. Afterwards, PEI-MAX was added, and the transfection mixture was mixed by inverting 5x. After an incubation phase of 20 minutes at room temperature, 1.5 mL of the transfection mixture was added per shake flask and the flask was placed back in the incubator.

Addition of enhancers:

Four to six hours after transfection, the one or more enhancer was added to the cell culture in the desired concentration. Subsequently, the culture was placed back in the incubator for further cultivation for three days at 37 °C, 5 % $CO_2$, and 130 rpm (FreeStyle 293-F) or 185 rpm (Viral Production Cells 2.0, LV MAX Production Cells and suspension-adapted HEK293 clone) at 50 mm orbit. Stock solutions of enhancers were pre-diluted in DMSO. Hence, the same volume of DMSO was added to all cultures, while concentrations of solubilized enhancers differed.

Harvest of rAAV from the cell culture:

After harvest, cells were chemically lysed with a detergent-based lysis buffer. To this end, 1 volume of lysis buffer was mixed with 9 volumes of cell culture and incubated for 2 hours at 37 °C on a shaking platform with 130 rpm and a deflection of 50 mm. The crude cell lysate was then spun down at 3,000 $x\,g$ for 10 min at 4 °C and was stored at -20 °C until further analysis.

Harvest of recombinant LV from cell culture:

The LV was harvested by centrifugation of the cell culture at 400 x g for 7 min followed by a 0.45 $\mu$m filtration step. The supernatant was stored at -20 °C until further analysis.

Quantification of rAAV genomes:

Briefly, sample preparation involved digesting free DNA by incubating the sample with DNase I (Thermo Fisher Scientific). DNase I was then inactivated by treatment with SDS (Thermo Fisher Scientific) and EDTA (Sigma Aldrich). Consequently, viral capsids were lysed by performing a proteinase K (Biotechrabbit) digest. Quantification of free rAAV genomes was performed on a QX200 ddPCR system (Bio-Rad) according to the manufacturer's protocol using an eGFP-specific primer/probe assay. The calculation of genomic titer was based on the following formula:

$$\text{vg per L} = \frac{22\,\mu L}{5.5\,\mu L} \times \text{copies per } \mu L \times 1e6\,\mu L \text{ per L} \times \text{dilution factor}$$

Quantification of LV genomes

To quantify the LV genomic titer, virus-containing supernatants were subjected to RNA extraction via the PureLink™ Viral RNA/DNA Mini Kit (Thermo Fisher Scientific), according to the kit manufacturer's protocol. Extracted RNA was incubated with DNase I. Afterwards, DNase I was inactivated by treatment with SDS (Thermo Fisher Scientific) and EDTA (Sigma Aldrich). Consequently, DNA-free RNA was transcribed into cDNA followed by ddPCR analysis using the One-Step RT-ddPCR Advanced Kit for Probes (Bio-Rad) according to the manufacturer's protocol and an eGFP-specific primer/probe assay. The calculation of genomic titer was based on the following formula:

$$\text{vg per L} = \frac{22\ \mu L}{5.5\ \mu L} \times \text{copies per } \mu L \times 1e6\ \mu L \text{ per L} \times \text{dilution factor}$$

Transduction assay of rAAV:

To quantify the concentration of transducing units in the rAAV-containing sample, a transduction assay was performed. Briefly, on the day of transduction, 100,000 cells in 1 mL of DMEM (+10 % FCS, + 2 mM L-glutamine) of adherent HEK293 (ATCC) were seeded in 12-well plates. Pre-dilutions of rAAV-containing samples were performed in PBS. Subsequently, 10 $\mu$L of either the undiluted or diluted sample was added to each well and cells were incubated for 72 h at 37 °C and 5 % CO2.

For the quantification of GFP positive cells, cells were washed once with PBS and trypsinated with 100 $\mu$L TrypLE Select per well. Trypsination was stopped by the addition of 900 $\mu$L DMEM containing 10 % FCS and 2 mM L-glutamine. Flow cytometry analysis was performed on an iQue® 3 flow cytometer system (Sartorius AG). GFP positive cells were gated above the 99 % interval of the negative (non-transduced) control. For the calculation of the transducing titer, dilutions with equal or less than 35 % positive cells were used. The calculations were based on the following formula:

$$\text{TU per L} = \frac{\% \text{ of GFP+ cells} \times 100{,}000 \text{ cells}}{10\ \mu L} \times 1e6\ \mu L \text{ per L}$$

Transduction assay of LV

To quantify the concentration of transducing units in the LV-containing sample, a transduction assay was performed. Briefly, on the day of transduction, 100,000 cells in 1 mL of DMEM/ F12 (+5 % FCS, + 6 mM L-glutamine, + 1.35 g/L glucose) of adherent HEK293FT containing 8 $\mu$g/mL hexadimethrine bromide were seeded in 12-well plates. Pre-dilutions of LV-containing samples were performed in PBS. Subsequently, 10 $\mu$L of either the undiluted or diluted sample was added to each well and cells were incubated for 72 h at 37 °C and 5 % $CO_2$. 24 h after transduction, a media change was performed to wash out hexadimethrine bromide.

For the quantification of GFP positive cells, cells were washed twice with PBS and trypsinated with 100 $\mu$L TrypLE Select per well. Trypsination was stopped by the addition of PBS containing 5 % FCS. Flow cytometry analysis was performed on an iQue® 3 flow cytometer system. Positive cells were gated above the 99 % interval of the negative (non-transduced) control. For the calculation of the transducing titer, dilutions with equal or less than 25 % positive cells were used. The calculations were based on the following formula:

$$\text{TU per L} = \frac{\% \text{ of GFP+ cells} \times 100{,}000 \text{ cells}}{10\ \mu L} \times 1e6\ \mu L \text{ per L}$$

**Example 1: Contacting mammalian cells with a flavonoid improved AAV production**

In Example 1, different HEK293 cell lines were transiently transfected to produce AAVs using the above-described protocols. After transfection, typically 4-6 hours after transfection, the HEK293 cells were contacted with a flavonoid, such as flavopiridol, and cultured in presence of the flavonoid to produce AAVs (see also schematic in Fig. 1). As demonstrated below, the flavonoid improved the AAV production. Specifically, the genomic viral vector titer but also the transducing viral vector titer was increased. Hence, it was shown that the amount of yielded AAVs was higher and that the AAVs were of higher quality in terms of transducing capacity.

Example 1A: Addition of flavopiridol increased the genomic titer of AAV in transiently transfected mammalian cells

In a first screening approach, FreeStyle 293-F cells (hereafter referred to as 2F) were transiently transfected in a 24-well plate according to the above-described protocol (see "Transient transfection of mammalian cells (HEK293) to produce rAAV in 24-well plates"). This protocol allows for testing a broad concentration range while limiting the use of materials.

The results from this first screening experiment showed a positive correlation of AAV2 genomic titer with increasing flavopiridol concentration compared to the DMSO control (see Figure 3). A nearly threefold, statistically significant increase in the genomic titer was achieved with the highest concentration of flavopiridol (50 nM). The results demonstrate that the addition of flavopiridol increased the genomic titer, wherein already low concentrations of 1 nM led to improvements and higher concentrations of 50 nM resulted in further improvements.

Example 1B: Addition of flavopiridol increased the genomic titer of AAV consistently for different mammalian cells

To test the scalability and reproducibility of the results of Example 1A, the next rAAV production process was carried out in 125 mL-shake flasks with two additional HEK293 cell lines, the Viral Production Cell 2.0 (hereafter referred to as PC) and a suspension-adapted HEK293 clone (hereafter referred to as HN). The latter one was cultured in HEK TF medium (Sartorius Xell GmbH), while the 2F and PC cell line were both cultivated in HEK ViP NB (Sartorius Xell GmbH). Because previous results indicated a positive correlation between AAV2 genomic titer and flavopiridol concentration, the highest concentration of 50 nM from the previous experiment was chosen for this example. It was expected that higher concentrations up to 100 nM would lead to a similar positive effect as observed previously for flavopiridol.

Overall, an increase in genomic titer for all cell lines tested was observed upon addition of 50 nM flavopiridol (Figure 4). Therefore, the beneficial effect seems to be independent of scale, culture vessel, culture medium and cell line. Interestingly, the relative increase of two- to threefold is remarkably similar to the relative increase achieved in well plates, demonstrating that the positive effect is independent of the scale. Nevertheless, AAV2 genomic titers were slightly increased when compared to the well plate screening. This is partly due to a higher viable cell density at the time of transfection (data not shown) and a higher amount of pDNA used, which are expectable side-effects the skilled person is aware of. Nevertheless, the beneficial effects of the flavonoid-compound flavopiridol is consistently demonstrated for different mammalian cell lines and at different scales.

Example 1C: Addition of flavopiridol increased the transducing titer of AAV consistently for different mammalian cells

An important quality parameter of rAAV preparations is the transducing or functional titer. Especially in clinical contexts, the ratio of packaged capsids and functional capsids becomes critical to prevent unwanted immune responses in the patient. Non-functional capsids essentially decrease the titer of a rAAV preparation and therefore make it necessary to increase the dose that is administered. A commercially relevant enhancer therefore should not only increase the genomic but also the transducing titer, at best in a similar manner. The addition of 50 nM flavopiridol five hours after transient transfection increased the transducing titer by nearly twofold for the 2F and PC cell lines, and the relative increase for the HN cells, although slightly lower, was also observable (Figure 5).

Hence, the flavonoid-enhancerflavopiridol advantageously increased the transducing titer, such that the obtained AAVs are more infective, i.e. these are of higher quality than AAVs obtained without flavopiridol (see control in Fig. 5).

Conclusions

Example 1 demonstrates that adding a flavonoid, such as flavopiridol, improves AAV production. Specifically, the inventors show that by contacting transiently transfected mammalian cells with flavopiridol, not only a higher genomic viral vector titer but also a higher transducing viral vector titer can be obtained. Thus, the yield of AAVs is higher and their quality for gene therapy applications.

The results further demonstrate that the advantageous properties of flavonoids, such as flavopiridol, are independent of the scale, as the positive effects were observed in well plate format but also for larger scale shake flask culture. Furthermore, by testing different mammalian cell lines, it was shown that the advantageous properties of the flavonoids are independent of the cell line, such that the flavonoids are applicable for different cell systems. In this respect, it should be noted that flavonoids, such as flavopiridol, can inhibit the cyclin-dependent kinase (CdK) and thus influence the cell cycle. As a result, it is believed that a higher percentage of cells remains in the S-phase, which allows for higher transcription rate and packaging efficiency of the viral vector. As it is believed that this could be a generic effect, the positive properties of flavonoids, such as flavopiridol, are not only useful for the tested transiently transfected HEK293 cells but it is expected that also other cell types, in particular mammalian cells which share important features in cell replication, as well as other production system, such as stably viral vector producing cells (see also schematic in Fig. 2) can also benefit from the described properties.

**Example 2: Contacting mammalian cells with M344 improved AAV production**

In Example 2, different HEK293 cell lines were transiently transfected to produce AAVs using the above-described protocols. After transfection, typically 4-6 hours after transfection, the HEK293 cells were contacted with M344, specifically at concentrations such as 2 $\mu$M but preferably higher than 5 $\mu$M, e.g. 10 $\mu$M and 20 pM, and cultured in presence of the M344 to produce AAVs (see also schematic in Fig. 1). As demonstrated below, the M344 improved the AAV production. Specifically, the genomic viral vector titer but also the transducing viral vector titer was increased. Hence, it was shown that the amount of yielded AAVs was higher and that the AAVs were of higher quality in terms of transducing capacity.

Example 2A: Addition of M344 increased the genomic titer of AAV in transiently transfected mammalian cells

In a first screening approach, 2F cells were transiently transfected in a 24-well plate according to the above-described protocol (see "Transient transfection of mammalian cells (HEK293) to produce rAAV in 24-well plates"). Thus, we were able to test a wider concentration range with less use of resources compared to shake flask processes. Previously performed experiments (not shown) indicated that concentrations up to 2 $\mu$M of M344 added to the culture result in a potential increase of genomic rAAV titer. Underscoring this, the results from this first screening experiment showed a positive correlation of AAV-2 genomic titer with increasing M344 concentration (Figure 6). We were able to achieve a nearly eightfold, statistically significant increase with the highest concentration of M344 (2 $\mu$M).

Example 2B: Addition of M344 increased the genomic titer of AAV consistently for different mammalian cells and at higher M344 concentrations.

Next, the concentration range of M344 was extended to include concentrations of 10 $\mu$M and 20 $\mu$M added to the culture five hours after transfection. Interestingly, the results indicate that the addition of 10 $\mu$M M344 led to an even higher increase of genomic AAV-2 titer (Figure 7). This is true for all three cell lines, therefore making the effect independent of cell lines, scale, culture vessel and culture medium. A nearly eightfold increase in the PC cell lines was achieved, while the 2F and HN cells showed a fivefold increase in genomic titer. Further increase in M344 concentration to 20 $\mu$M, led to a titer which is still significantly higher than 2 $\mu$M but slightly lower than for 10 $\mu$M.

Example 2C: Addition of M344 increased the transducing titer of AAV consistently for different mammalian cells

According to our results, the positive effect of M344 on the genomic titer is also reflected in the transducing titer (see Figure 8). The addition of 10 $\mu$M led to a nearly tenfold, statistically significant increase in the transducing titer in PC cells when compared to the control. For the two other cell lines, 2F and HN, the increase was nearly four- to fivefold and statistically significant. As was the case with the genomic titer, further increasing the concentration of M344 to 20 $\mu$M decreased the transducing titer compared to 10 $\mu$M but still led to a higher transducing titer than for 2 $\mu$M M344. Overall, the effect of M344 on the transducing titer is remarkably like its effect on genomic titer.

Conclusions

Example 2 demonstrates that adding M344, particularly in concentrations above 2 $\mu$M, preferably higher than 5 $\mu$M, such as 10 $\mu$M and 20 $\mu$M, improves AAV production. Specifically, the inventors show that by contacting transiently transfected mammalian cells with M344 a higher genomic viral vector titer but also a higher transducing viral vector titer can be obtained. Thus, the yield of AAVs is higher but also their quality for gene therapy applications.
The results further demonstrate that the advantageous properties of M344 are independent of the scale, as the positive effects were observed for a well plate format but also for larger scale shake flask culture. Furthermore, by testing different mammalian cell lines, it was shown that the advantageous properties of M344 are independent of the cell line, such that the M344 is applicable for different cell systems. In this respect, it should be noted that M344, is a potent histone deacetylase (HDAC) inhibitor with a high selectivity for HDAC6. This class of HDAC is involved in the clearance of ubiquitinated proteins. Also, it is hypothesized that a hindered clearance of rAAV capsid proteins leads to an overall higher packaging efficiency and therefore a higher genomic and functional titer. As it is believed that this could be a generic effect the positive properties of M344 are not only useful for the tested transiently transfected HEK293 cells but it is expected that also other cell types, in particular mammalian cells which share important features in cell replication, as well as other production system, such as stably viral vector producing cells (see also schematic in Fig. 2) can benefit from the described properties.

**Example 3: Contacting mammalian cells with M344 improved lentivirus production**

In Example 3, LV MAX production cells were transiently transfected to produce lentiviruses (LVs) using the above-described protocols. After transfection, typically 4-6 hours after transfection, the LV MAX production cells were contacted with M344, specifically at concentrations such as 2 $\mu$M but preferably higher than 5 $\mu$M, e.g. 8 $\mu$M, 10 $\mu$M and 15

pM, and cultured in presence of the M344 to produce lentiviruses (see also schematic in Fig. 1). As demonstrated below, the M344 improves the lentivirus production. Specifically, the genomic viral vector titer but also the transducing viral vector titer is increased. Hence, it is shown that the amount of yielded lentiviruses is higher and that the lentiviruses are of higher quality in terms of transducing capacity.

Specifically, LV Max Production cells were transfected in shake-flask scale and M344 was added four to six hours post transfection in concentrations ranging between 0.5 to 15 $\mu$M. As a control an untreated sample, as well as a sample containing 0.6% DMSO was used. As shown in Figure 9, concentrations of 0.5 and 2.5 $\mu$M result in only a small increase in genomic titer compared to the untreated and DMSO control, whereas a concentration of 5 $\mu$M increased the genomic titer by a factor of ~2.5. A further increase in M344 concentration up to 8, 10 and 15 $\mu$M results in the same titer increase as 5 $\mu$M. Matching observations can also be obtained from the transducing titers (see Fig. 10): Only minor increase in the transducing titer can be observed after addition of 0.5 and 2.5 $\mu$M M344, whereas M344 concentrations of 5 to 15 $\mu$M led to a -2-fold and ~2.5 increase in transducing titer, respectively.

Example 3 demonstrates that adding M344, particularly in high contractions of 5 $\mu$M and higher improved LV production. Specifically, the inventors show that by contacting transiently transfected mammalian cells with M344 a higher genomic viral vector titer but also a higher transducing viral vector titer can be obtained. Thus, the yield of LVs is higher but also their quality for gene therapy applications.

**Example 4: Contacting mammalian cells with a flavonoid-enhancer and M344 improved AAV production**

In Example 4, different HEK293 cell lines were transiently transfected to produce AAVs, using the above-described protocols. After transfection, typically 4-6 h after transfection, HEK293 cells were contacted with a combination of (i) a flavonoid, such as flavopiridol, (ii) M344 and (iii) sinapinic acid at different concentrations and cultured in the presence of these compounds to produce AAVs (see also schematic in Figure 1). In particular, M344 was added in concentrations between 0.3 and 3 $\mu$M, flavopiridol was added in concentrations between 1 and 50 nM and sinapinic acid was added in concentrations between 0.05 and 0.75 mM. Sinapinic acid was believed to further improve AAV production, however, results indicate that sinapinic acid had no or in higher concentrations even negative effects (not shown). As demonstrated below, the combinations improved AAV production. Specifically, the genomic viral vector titer but also the transducing viral vector titer were increased. Hence, it was shown that the amount of yielded AAVs was higher and that the AAVs were of higher quality in terms of transducing capacity.

Example 4A: Combinatorial addition of flavopiridol and M344 enhanced AAV production in different cell lines more than addition of single compounds

Because flavopiridol and M344 have both proven to increase rAAV genomic titer and transducing titer, both were tested in combination with sinapinic acid. The compounds were added five hours after transient transfection. The results as shown in Fig. 11 underscore the previous results, wherein flavopiridol and M344 both led to considerable increases in AAV2 genomic titer in all cell lines tested.

On the other hand, this example revealed an additive effect of flavopiridol and M344 in their highest concentrations (50 nM and 3 $\mu$M, respectively) on rAAV genomic titer in all three cell lines.

The 2F cells exhibited a four-fold increase in genomic titer, while an eight-fold increase was observed for the PC cell line. The effect in the HN cell lines mounted to a five-fold increase in genomic titer. Sinapinic acid was added in the same concentrations in all experiments, thereby exhibiting no obvious effect, whether it might have been positive or negative. In other experiments (not shown here), increased concentrations of sinapinic acid indeed had a negative effect on genomic titer, such that it is believed that the genomic titer is even higher without sinapinic acid.

Further combinations were tested in the different cell lines (see Figs. 12A for 2F, 12B for PC), wherein low concentrations of M344 and/or flavopiridol (such as #1: 0.3 $\mu$M M344, 1 nM flavopiridol and 0.05 mM sinapinic acid or #2: 3 $\mu$M M344, 1 nM flavopiridol and 0.05 mM sinapinic acid) already showed a marked increase. Increasing the concentrations even more (such as #3: 0.3 $\mu$M M344, 50 nM flavopiridol and 0.05 mM sinapinic acid or #4: 3 $\mu$M M344, 50 nM flavopiridol and 0.05 mM sinapinic acid) increased the genomic titer further. While differences between the two cell lines are visible, it is remarkable that for each compound M344 and flavopiridol positive effects compared to the control are visible and further enhancements are achieved by higher concentrations. In some cases, these beneficial effects go beyond additive effects such that synergistic effects were observed.

Example 4B: Combinatorial addition of flavopiridol and M344 enhanced transducing titer of AAV preparations in different cell lines more than addition of single compounds

Based on the findings from Example 4A, it was of particular interest to evaluate the combinatorial effect of flavopiridol, M344 and sinapinic acid on the biological activity of the produced rAAVs. Therefore, the transducing titer was measured, as shown in Fig. 13. According to the results, the effect of the combination of flavopiridol and M344 in their highest concentrations led to a five-fold increase in transducing titer in the 2F cells when compared to the control (see Fig. 13). The HN cells showed a near two-fold increase. The most profound effect was seen for the PC cells, where the combination of flavopiridol and M344 achieved a 32-fold increase in transducing titer.

Conclusion

Example 4 demonstrates that adding a flavonoid-enhancer such as flavopiridol in combination with M344, particularly in concentrations of 50 nM and 3 $\mu$M, respectively, improved AAV production. Specifically, the inventors show that by contacting transiently transfected mammalian cells with a combination of flavopiridol and M344 a higher genomic viral vector titer but also a higher transducing viral vector titer can be obtained when compared to the addition of each compound separately. Thus, the yield of AAVs is higher but also their quality for gene therapy.

Furthermore, by testing different mammalian cell lines, it was shown that the advantageous properties of the combinatorial addition of flavopiridol and M344 are independent of the cell line or cell type, such that this strategy is applicable for different cell systems. Noteworthy, the addition of sinapinic acid did not have any effect or showed in some experiments negative effects (now shown). It could be that the results of Example 4 would be even higher if sinapinic acid would not have been added.

In this respect, it should further be noted that the results suggest that flavopiridol and M344 affected different pathways or act on different mechanisms, and these effects did not block each other, highlighting the potential of the presented combinatorial approach, also for other flavonoids in combination with M344. Furthermore, M344 inhibits the clearance of ubiquitinated proteins such as viral capsid proteins, and flavopiridol leads to a cell cycle arrest and has a global effect on cellular protein synthesis. Thus, the combinatorial approach could help at syncing the onset of viral genome replication and the assembly and supply of viral capsids, consequently increasing the packing efficiency. The results indicate that the combination of M344 and flavopiridol is not only useful for the tested cell lines but various cell systems but also production systems.

**Claims**

1. A viral vector production method, comprising following steps:

    (a) providing a mammalian cell configured to produce a viral vector;
    (b) contacting the mammalian cell with a flavonoid and/or M344;
    (c) culturing the mammalian cell in presence of the flavonoid and/or M344 to produce a viral vector; and
    (d) optionally, harvesting the viral vector.

2. The method according to claim 1, wherein the flavonoid is a flavone, preferably flavopiridol or a derivative thereof.

3. The method according to claim 1 or 2, wherein the flavonoid inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof, more preferably inhibiting at least CdK9.

4. The method according to one or more of claims 1 to 3, wherein step (b) comprises contacting the mammalian cell with the flavonoid resulting in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM in a composition with the mammalian cell.

5. The method according to one or more of claims 1 to 4, wherein step (b) comprises contacting the mammalian cell with the M344 resulting in a concentration of at least 0.5 $\mu$M, at least 1 $\mu$M, at least 1.5 pM, at least 2 pM, at least 2.5 pM, at least 3 pM, at least 4 $\mu$M, at least 5 $\mu$M, at least 6 pM, at least 7 pM, at least 8 pM, at least 9 pM, at least 10 $\mu$M, preferably at least 5 $\mu$M in a composition with the mammalian cell.

6. The method according to one or more of claims 1 to 5, wherein the genomic viral vector titer and/or transducing viral vector titer is increased compared to a viral vector production method without contacting the mammalian cell with the flavonoid and/or the M344, preferably wherein:

    - the genomic viral vector titer is increased by at least 10%, preferably at least 25%, compared to a viral vector production method without contacting the mammalian cell with the flavonoid and/or the M344; and/or

- the transducing viral vector titer is increased by at least 10%, preferably at least 25%, compared to a viral vector production method without contacting the mammalian cell with the flavonoid and/or M344.

7. The method according to one or more of claims 1 to 6, wherein the viral vector is a lentivirus or an adeno-associated virus (AAV).

8. The method according to one or more of claims 1 to 7, wherein the mammalian cell is modified to be configured to produce a viral vector, preferably by transiently transfecting the mammalian cell with one or more plasmids for viral vector production allowing for transient viral vector production, preferably wherein transiently transfecting comprises:

(x) combining the mammalian cell with the one or more plasmids for viral vector production and a transfection reagent; and
(y) incubating the mammalian cell, the one or more plasmids for viral vector production and the transfection reagent to generate a transiently transfected cell.

9. The method according to claim 8, wherein step (y) comprises incubating for at least 1 hour, at least 2 hours, at least 3 hours, preferably at least 4 hours.

10. The method according to one or more of claims 1 to 9, wherein the mammalian cell is selected from a HEK293 cell or a derivative of a HEK293 cell, preferably adapted for suspension culture.

11. The method according to one or more of claims 1 to 10, wherein the method has one or more of the following characteristics:

- step (a) further comprises culturing the mammalian cell in suspension culture;
- step (c) comprises culturing the mammalian cell in suspension culture;
- the viral vector is harvested in step (d), preferably wherein harvesting comprises lysing the transiently transfected cells;
- it further comprises step (e) purifying the harvested viral vector; and/or
- it further comprises step (f) formulating the viral vector for gene therapy.

12. A viral vector production system, comprising:

- a mammalian cell configured to transiently or stably produce a viral vector, preferably the mammalian cell is a HEK293 cell or a derivative of a HEK293 cell; and
- a flavonoid and/or M344; and
- a cell culture medium for culturing the mammalian cell.

13. A viral vector production composition, comprising:

- a mammalian cell configured to transiently or stably produce a viral vector, preferably the mammalian cell is a HEK293 cell or a derivative of a HEK293 cell; and
- a flavonoid and/or M344;

wherein the flavonoid and/or M344 increases production of the genomic viral vector titer and/or transducing viral vector titer compared to a composition without the flavonoid and/or M344.

14. The system according to claim 12 or the composition according to claim 13, wherein the flavonoid has one or more of the following characteristics:

- it is a flavone, preferably flavopiridol or a derivative thereof;
- it inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof;
- it inhibits at least CdK9; and/or
- it is present in a concentration of at least 1 nM, at least 2 nM, at least 3 nM, at least 4 nM, at least 5 nM, at least 6 nM, at least 7 nM, at least 8 nM, at least 9 nM, at least 10 nM, at least 15 nM, at least 20 nM, at least 25 nM, at least 30 nM, at least 35 nM, preferably, at least 40 nM, at least 45 nM or at least 50 nM.

15. The system according to claim 12 or 14 or the composition according to claim 13 or 14, wherein the M344 is present in a concentration of at least 0.5 pM, at least 1 pM, at least 1.5 pM, at least 2 pM, at least 2.5 pM, at least 3 pM, at least 4 pM, at least 5 pM, at least 6 pM, at least 7 $\mu$M, at least 8 pM, at least 9 pM, at least 10 $\mu$M, preferably at least 5 $\mu$M.

16. Flavonoid for use for increased transient or stable viral vector production.

17. The flavonoid for use according to claim 16, wherein the flavonoid

   - is a flavone, preferably flavopiridol or a derivative thereof; and/or
   - inhibits one or more cyclin-dependent kinases (CdK), preferably selected from CdK1, CdK2, Cdk3, CdK4, CdK5, CdK6, CdK7, CdK8, CdK9, CdK10, or CdK11 or a combination thereof.

Figure 1

| Mammalian cell in different bioprocess scales | Transient transfection | Increased production of viral vector | Several fold increase in yield |

Figure 2

| Mammalian cell in different bioprocess scales | Use of mammalian cells with stably integrated viral vector genes | Increased production of viral vector | Several fold increase in yield |

Figure 3

Figure 4

Figure 5

Figure 6

Figure 7

Figure 8

Figure 9

Figure 10

Figure 11

Figure 12

A

| | 1 | 2 | 3 | 4 | Control |
|---|---|---|---|---|---|
| M344 in µM | 0.3 | 3 | 0.3 | 3 | |
| Flavopiridol in nM | 1 | 1 | 50 | 50 | |
| Sinapinic acid in mM | 0.05 | 0.05 | 0.05 | 0.05 | |

B

| | 1 | 2 | 3 | 4 | Control |
|---|---|---|---|---|---|
| M344 in µM | 0.3 | 3 | 0.3 | 3 | |
| Flavopiridol in nM | 1 | 1 | 50 | 50 | |
| Sinapinic acid in mM | 0.05 | 0.05 | 0.05 | 0.05 | |

Figure 13

Europäisches
Patentamt

European
Patent Office

Office européen
des brevets

**EUROPEAN SEARCH REPORT**

Application Number

EP 23 20 0346

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCARROTT JOSEPH M. ET AL: "Increased recombinant adeno-associated virus production by HEK293 cells using small molecule chemical additives", BIOTECHNOLOGY JOURNAL, vol. 18, no. 3, 22 December 2022 (2022-12-22), XP093080843, DE ISSN: 1860-6768, DOI: 10.1002/biot.202200450 Retrieved from the Internet: URL:https://onlinelibrary.wiley.com/doi/full-xml/10.1002/biot.202200450> [retrieved on 2024-03-14] p. 2, column 1, paragraph 3,p. 2, column 2, paragraph 2, p. 3, column 2 paragraphs 2 - 3,p.6, column 2, paragraph 3 - p. 7; * abstract; figures 1,5; table S1 * | 1,5-13, 15 | INV. C12N15/86 |
| X | CN 112 980 884 A (BEIJING CHINAGENE TECH CO LTD) 18 June 2021 (2021-06-18) * paragraphs [0004-0043], [0080-0081], [0125], [0131-0168]; claims 1-5,8-10; figures 10-17 * | 1-4, 6-14,16, 17 | TECHNICAL FIELDS SEARCHED (IPC) C12N |
| X | WO 2022/159702 A1 (BIOGEN MA INC [US]) 28 July 2022 (2022-07-28) * paragraphs [2-5], [13],[32], [21-22],[179-184],[122]; claims 1, 9-11, 16-18, 20, 24-26; figures 1-2; example 1 * | 1-3, 6-14,16, 17 | |

-/--

The present search report has been drawn up for all claims

| Place of search | Date of completion of the search | Examiner |
|---|---|---|
| Munich | 19 March 2024 | Empl, Laura |

Europäisches
Patentamt

European
Patent Office

Office européen
des brevets

# EUROPEAN SEARCH REPORT

**Application Number**

EP 23 20 0346

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PINTO NICOLE ET AL: "Flavopiridol causes cell cycle inhibition and demonstrates anti-cancer activity in anaplastic thyroid cancer models", PLOS ONE, vol. 15, no. 9, 24 September 2020 (2020-09-24), page e0239315, XP093142145, US ISSN: 1932-6203, DOI: 10.1371/journal.pone.0239315 p.2, paragraph 3; * abstract * | 3,17 | |
| A | SEDLACEK H H ED – NIESVIZKY DR RUBEN: "MECHANISMS OF ACTION OF FLAVOPIRIDOL", CRITICAL REVIEWS IN ONCOLOGY/HEMATOLOGY, ELSEVIER, AMSTERDAM, NL, vol. 38, no. 2, 1 January 2001 (2001-01-01), pages 139-170, XP009034115, ISSN: 1040-8428, DOI: 10.1016/S1040-8428(00)00124-4 * abstract * | 1-17 | |
| A | SAISOMBOON SAOWALUK ET AL: "Antitumor effects of flavopiridol, a cyclin-dependent kinase inhibitor, on human cholangiocarcinoma in vitro and in an in vivo xenograft model", HELIYON, vol. 5, no. 5, 1 May 2019 (2019-05-01), page e01675, XP093142147, GB ISSN: 2405-8440, DOI: 10.1016/j.heliyon.2019.e01675 p.2; * abstract; figure 1 * | 4 | TECHNICAL FIELDS SEARCHED (IPC) |

The present search report has been drawn up for all claims

| Place of search | Date of completion of the search | Examiner |
|---|---|---|
| Munich | 19 March 2024 | Empl, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 530 353 A1

## ANNEX TO THE EUROPEAN SEARCH REPORT
## ON EUROPEAN PATENT APPLICATION NO.

EP 23 20 0346

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2024

| Patent document cited in search report | | Publication date | Patent family member(s) | | Publication date |
|---|---|---|---|---|---|
| CN 112980884 | A | 18-06-2021 | NONE | | |
| WO 2022159702 | A1 | 28-07-2022 | CA | 3207895 A1 | 28-07-2022 |
| | | | CN | 116917467 A | 20-10-2023 |
| | | | EP | 4281550 A1 | 29-11-2023 |
| | | | JP | 2024504365 A | 31-01-2024 |
| | | | WO | 2022159702 A1 | 28-07-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023139224 A
- WO 2020033842 A1

- WO 2007127264 A2

**Non-patent literature cited in the description**

- **ROLDAO et al.** *Comprehensive Biotechnology*, 2017, vol. 1, 633-656
- **PANCHE et al.** *Journal of Nutritional Science*, 2016, vol. 5 (e47), 1-15
- **JAIN et al.** *Mini-Reviews in Medicinal Chemistry*, 2012, vol. 12, 1-18

- **KNOCHE et al.** *pLoS ONE*, 2022, vol. 17 (9), e0273518
- **KNOCHE et al.** *PLoS ONE*, 2022, vol. 17 (9), e0273518